# EUROPEAN PATENT SPECIFICATION

(11) **EP 3 946 733 B1**
(45) Date of publication and mention of the grant of the patent: **08.05.2024**
(21) Application number: 20712826.5
(22) Date of filing: 30.03.2020
(51) Int. Cl.: B01L 3/00, B01L 3/02, C12M 1/34, C12M 1/42, C12M 3/06

(54) **A METHOD AND DEVICE FOR TRANSFECTING CELLS**
VERFAHREN UND VORRICHTUNG ZUR TRANSFEKTION VON ZELLEN
PROCÉDÉ ET DISPOSITIF DE TRANSFECTION DE CELLULES

(30) Priority: 29.03.2019 EP 19166409; 19.06.2019 EP 19181333
(43) Date of publication of application: 09.02.2022
(73) Proprietor: Cellix Limited, D12 EK79 Dublin (IE)
(72) Inventor: KASHANIN, Dmitry, 18 Dublin (IE); WILLIAMS, Vivienne, 3 Dublin (IE); SHVETS, Igor, 15 Dublin (IE)
(74) Representative: Purdylucey Intellectual Property
(86) International application number: PCT/EP2020/058939
(87) International publication number: WO 2020/201206

(56) References cited:
- US-A1- 2007 155 016
- US-A1- 2019 119 624
- Sebastian C Bürgel ET AL: "COMBINED MICROFLUIDIC SINGLE-CELL ELECTROPORATION AND IMPEDANCE SPECTROSCOPY ANALYSIS", , 28 October 2012 (2012-10-28), pages 410-412, XP055639925, Retrieved from the Internet: URL:https://www.rsc.org/images/loc/2012/pd f/M.2.43.pdf [retrieved on 2019-11-07]
- BÜRGEL SEBASTIAN C ET AL: "On-chip electroporation and impedance spectroscopy of single-cells", SENSORS AND ACTUATORS B: CHEMICAL, vol. 210, 17 December 2014 (2014-12-17), pages 82-90, XP029141593, ISSN: 0925-4005, DOI: 10.1016/J.SNB.2014.12.016
- XIAOLIANG GUO ET AL: "Controllable in-situ cell electroporation with cell positioning and impedance monitoring using micro electrode array", SCIENTIFIC REPORTS, vol. 6, no. 1, 10 August 2016 (2016-08-10) , XP055640950, DOI: 10.1038/srep31392
- MINGDE ZHENG ET AL: "Continuous-flow, electrically-triggered, single cell-level electroporation", TECHNOLOGY, vol. 05, no. 01, 1 March 2017 (2017-03-01) , pages 31-41, XP055640954, ISSN: 2339-5478, DOI: 10.1142/S2339547817500017

## Description

### Field of the Invention

The present invention relates to a method and system for treatment of cells, and to a method and system for electroporation and/or transfection of cells.

### Background to the Invention

In the past decade genetic engineering of living cells and organisms has become a hugely important field of research with exciting applications envisaged in medicine, farming, production of animals, production of food and other areas. The momentum in this field has accelerated with the advent of the CRISPR/Cas9 genome-editing platform.

In these methods it is essential to deliver biological material (e.g. DNA, RNA, gRNA, ribonucleoprotein (RNP), protein, virus, etc) from the outside of the cell across the cell membrane to the interior of the cell, this process is called transfection. It is important that the cell stays alive after the completion of the transfection to initiate the induction of the transfected material within the cell's genome. Therefore, developing methods of transfection has gained much importance in recent years.

Generally, the cell membrane protects interior of the cell from the introduction of alien biological material (i.e. transfecting the cell) and therefore the transfection must disrupt temporarily this barrier function of the membrane. There are several ways of transfecting a cell including using chemical transfection method, cell squeezing and mechanical processing of cells in a mix with beads. Electroporation appears to be the most commonly used method for transfection. Electroporation occurs when the living cell is exposed to an external electric field, making the transmembrane potential exceed a critical threshold value. This leads to the creation of nanoscale pores in the cell membrane, thus making it transiently and reversibly permeable. Electroporation and cell transfection performed in a microfluidic channel has been described previously.

US2019/119624 describes a microfluidic cell transfection device having an upstream sensor and an electroporation electrode assembly downstream of the sensor, and an acousto-phoretic cell alignment system that aligns cells in the centre of the channel. The sensor can detect the size of passing cells based on changes in current in the microfluidic channel and then adjust the electroporation voltage accordingly. It cannot provide for on-demand actuation of the electroporation voltage, meaning that the voltage is continuously applied across the microfluidic channel. This can cause changes in the pH of the carrier fluid in the microfluidic channel and decrease sensitivity and accuracy. In addition, the electroporation electrodes will not have a long life.

Burgel et al (SENSORS AND ACTUATORS B: CHEMICAL Vol. 201; 1 April 2015) describes a research tool for studying the effects of voltage on permeability of cells. Cells are passed along a microfluidic channel having detection electrodes upstream of electroporation electrodes. The two detection electrodes detect electrical impedance (EIS) changes in cells after they have been electroporated, by shuttling the cells back and forth between the detection and electroporation electrodes. It is not a continuous system and is therefore not suitable for high-throughput electroporation and transfection of a population of cells.

Guo et al. (SCIENFITIC REPORTS, Vol. 6, No. 1, 10 August 2016) describes a microfluidic system having a cell positioning module, electroporation module, and downstream electrical impedance measurement module. Cells are delivered into a chip array and seeded at positions between the electrodes in dielectrophoretic traps, where electroporation and analysis is performed in static conditions. Therefore it is a slow, non-continuous system, and not suitable for high-throughput cell transfection and analysis.

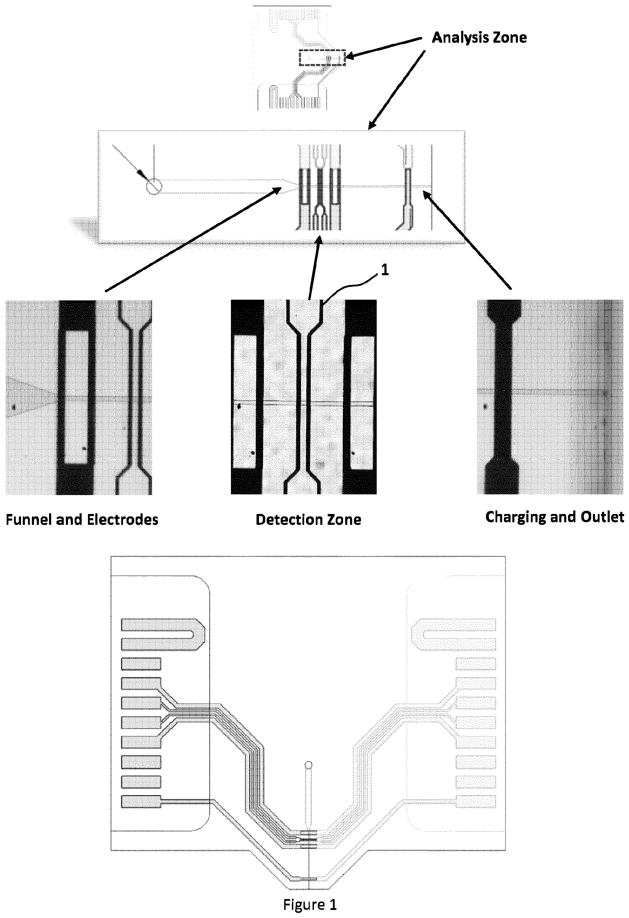

Zheng et al. (TECHNOLOGY, Vol. 10, No. 1, 1 March 2017) describes a continuous system for transfection of cells. The system employs electrodes at each end of a constricted microfluidic channel and employs a continuous electric field along the channel which detects when a cell enters the constricted zone and then actuates the electroporation voltage along the channel. The same electrodes are employed for detection and electroporation, and this requires the input of the lock-in amplifier to be disconnected from the electrodes so that it does not overload when a pulse of electroporation voltage is applied to the electrodes. It is not possible to connect and disconnect a lock-in amplifier input for a fast continuous sensitive operation. Furthermore, this needs to be done with a mechanical switch such as relay as transistors would not provide sufficient voltage isolation (the mechanical switch is shown in Fig. 1c). The net result is that this is a very slow, non-high-throughput, process which is evidenced by the stated rate of cell transfection of 1.3 cells/second.

Burgel et al. ("Combined Microfluidic Single-Cell Electroporation and Impedance Spectroscopy Analysis", 28 October 2012, pages 410-412, XP055639925) discloses a microfluidic system for single-cell electroporation and label-free assessment of the process through upstream electrical impedance spectroscopy (EIS). A syringe pump is used to shuttle a cell back and forth an EIS analysis region. Each time a cell passes the measurement electrodes, the impedance spike of the cell is recorded and uses to generate a field strong enough to electroporate the cell. The system is a slow, non-continuous, system.

It is an object of the invention to overcome at least one of the above-referenced problems. It is a particular object of the invention to provide a continuous high-throughput method of electroporation and transfection of a large population of cells suitable for use in a commercial setting.

### Summary of the Invention

The Applicant has addressed the problems of the prior art by providing a continuous high-throughput system and method for treatment (e.g. electroporation and transfection) of cells as set out in the appended claims. In one aspect, the system employs a detection zone to detect the velocity of cells, a cell treatment (e.g. electroporation) zone downstream of the detection zone, and a processor configured to transiently actuate the cell treatment module (e.g. electroporation electrodes) in the cell treatment (e.g. electroporation) zone based on the determined velocity of the cells and the distance between the cell treatment zone and the detection zone. In the case of a system for electroporation of cells, this allows the electroporation voltage to be applied only when cells are passing the electroporation zone, and avoids voltage-induced changes to the pH of the carrier fluid that is detrimental to the recovery of the cells after the electroporation, and excessive wear on the electrodes. It also allows the duration of the electroporation pulse to be varied according to the level of electroporation required, i.e. the pulse may be applied for the duration of the time the selected cell is passing the electroporation electrodes, or for only a part of the time that the cell passes the electrodes, or two or more separate pulses may be applied. Generally, the pulse is a pulse of AC voltage applied to the electroporation electrodes. The system and method of the invention may be applied to control microfluidic treatments of cells other than electroporation (for example cell sorting, cell manipulation, cell deflection) where accurate timing of the arrival and residence time of a cell at a treatment zone may be required).

In a first aspect, the invention provides a system for continuous high-throughput treatment of a population of cells as set out in Claim 1.

Generally, the cell treatment may be any physical or electrical manipulation of the cell, i.e. charging the cell, electroporation of a cell, movement of a cell. In a preferred embodiment, the cell treatment is cell electroporation and optionally cell transfection.

In any embodiment, the detection electrode module comprises a first detection electrode pair configured to detect a first change in electrical impedance across the microfluidic channel corresponding to the cell passing the first detection electrode pair and a second detection electrode pair configured to detect a second change in electrical impedance across the microfluidic channel corresponding to the cell passing the second detection electrode pair, wherein the processor is configured to analyse the detected first and second changes in electrical impedance and determine the time period T1 it takes for the cell to pass from between the first and second detection electrode pairs, and calculate the velocity V of the cell based on the time period T1 and the distance D1 between the first and second detection electrode pairs.

In any embodiment, the processor is configured to transiently actuate the cell treatment module for a treatment period T2 corresponding to the cell passing all or part of the cell treatment module.

Generally, the at least one detection electrode pair of a detection electrode module comprises at least one excitation electrode connected to at least one AC voltage source, and at least one detection electrode connected to at least one AC detection circuit.

In any embodiment, the cell treatment module comprises a cell electroporation module configured to electroporate cells passing the cell electroporation module by passing a cell electroporation voltage across the microfluidic channel.

In any embodiment, the processor is configured to compare the change in electrical impedance detected by the detection electrode module corresponding to a cell passing the upstream detection zone module with a reference change in electrical impedance, calculate a cell electroporation parameter selected from amplitude of voltage or number of duration of the electroporation pulse based on the comparison, and actuate the cell electroporation module to apply the electroporation parameter to the cell. Thus, the system can detect the baseline level of the electrical characteristics of the cells and adjust the electroporation parameters accordingly. Thus, for example, if the change in electrical impedance in the detection zone is between X1 and X2, the amplitude of the electroporation voltage may be set at V1, and if the if the change in electrical impedance in the detection zone is between X3 and X4, the amplitude of the electroporation voltage may be set at V2.

In any embodiment, the system comprises a hydrodynamic cell focussing apparatus fluidically coupled to the microfluidic channel and configured to provide a focussed stream of cell-containing fluid upstream of the upstream detection zone. The focussed stream generally comprises a core cell-containing carrier fluid stream and a positioning stream of fluid forming a sheath around the core stream upstream of the electroporation zone. The advantage of using hydrodynamic focussing is that all the cells pass through the detection zone at the same distance from the detection electrodes. This improves the accuracy of detection of the cell status. Furthermore, by the same token the cells then pass through the electroporation zone at the same distance from the electroporation electrodes. This improves the consistency of electroporation across a population of cells.

In any embodiment, the cell hydrodynamic focussing apparatus is configured to focus the single train of cells asymmetrically in the microfluidic channel so that cells are positioned closer to one electrode of a detection electrode module than a second electrode of the detection module. The Applicant has discovered that positioning the cells closer of one electrode of a detection electrode pair provides for better detection sensitivity and selectivity. The methods and devices for forming a train of cells, making the cells pass through a desired location within the cross-section of the microfluidic channel, and alignment of the cells are explained in European patent applications [EP 17177619.8-1553 "A microfluidic chip";
EP 17177631.3-1553, "Apparatus and Method for Improved Identification of Particles and Cells";
EP 16166177.2-1371 "A microfluidic chip for focussing a stream of particulate containing fluid";
EP 17177662.8-1553, "System and Method for Improved Identification of Particles or Cells";
EP 17177624.8-1553, "A microfluidic apparatus for separation of particulates in a fluid"; PCT Application No. PCT/EP2017/062574, "AN APPARATUS FOR MICROFLUIDIC FLOW CYTOMETRY ANALYSIS OF A PARTICULATE CONTAINING FLUID"].

In any embodiment, the system comprises a downstream detection zone disposed downstream of the cell treatment zone and comprising a detection electrode module configured to detect a downstream change in electrical impedance across the microfluidic channel at the downstream detection zone corresponding to a cell passing the downstream detection zone, wherein the processor is operatively coupled to the second detection electrode module.

In any embodiment, the processor is configured to compare the downstream change in electrical impedance corresponding to a cell passing the downstream detection zone with a reference change in electrical impedance corresponding to a known cell electroporation status, and determine electroporation status of the cell based on the comparison.

In any embodiment, the processor is configured to compare the downstream change in electrical impedance corresponding to a cell passing the downstream detection zone with an upstream change in electrical impedance detected by the upstream detection zone corresponding to the same cell passing the upstream detection zone, and determine electroporation status of the cell based on the comparison.

The processor may be configured to determine an electroporation status of a single cell selected from cell viability, successful cell electroporation, unsuccessful cell electroporation.

In any embodiment, the processor is configured to compare the downstream change in electrical impedance corresponding to a cell passing the downstream detection zone with a reference change in electrical impedance corresponding to a known cell electroporation status, and then actuate the cell electroporation module to modify the cell electroporation voltage or the duration of the cell electroporation voltage pulse applied across the microfluidic channel based on the comparison if required.

In any embodiment, the processor is configured to compare the downstream change in electrical impedance corresponding to a cell passing the downstream detection zone with an upstream change in electrical impedance detected by the upstream detection zone corresponding to the same cell passing the upstream detection zone, and actuate the cell electroporation module to modify the cell electroporation voltage or the cell electroporation voltage pulse applied across the microfluidic channel based on the comparison if required.

Thus, changes in electrical impedance across the microfluidic channel corresponding to a passing cell may be used to determine transfection status of the cell or as a means of monitoring and if required modifying the electroporation voltage or duration or number of pulses of the electroporation voltage. Preferably, the processor is configured to determine a baseline change in electrical impedance for a cell (upstream detection) and then a change in electrical impedance after electroporation, and determine based on the two values whether the permeability of the cell membrane has been sufficiently modified to allow transfection. For example, if it is determined that a cell is not transfection competent after electroporation, the processor may increase the electroporation voltage and pulse duration. Likewise, if a cell is determined to be non-viable, the electroporation voltage or duration of pulse may be decreased. In this manner, the system can dynamically modify the electroporation parameters in real-time to adjust the system to achieve continuous, high-throughput successful cell electroporation and transfection.

In any embodiment, the fluidic device comprises a cell separation module downstream of the downstream detection zone, wherein the cell separation module is operatively coupled to the processor and comprises a force generator configured to separate single selected cells from the population of cells based on the electroporation (or treatment) status of the cell determined by the processor. Cells that are detected to be non-viable or transfection incompetent (or both) may be separated from the population of cells using a cell separation module as described below.

In any embodiment, the processor is configured to calculate a time T2 it takes for a cell to travel from a detection electrode module (generally the upstream detection electrode module) to the separation module based on the determined velocity V and distance D2 between the detection electrode module and the separation module, and actuate the separation module a time T2 after the cell passes the detection electrode module. In any embodiment, the processor is configured to calculate a time T2 it takes for a cell to travel from the electroporation electrode module to the separation module based on the determined velocity V and distance D2 between the electroporation electrode module and the separation module, and actuate the separation module a time T2 after the cell passes the electroporation electrode module.

In one embodiment the separation module comprises a force generator configured to deliver a pulse of force to passing cells individually, one cell at a time. This could be done by using the method described in [EP 17177624.8-1553, "A microfluidic apparatus for separation of particulates in a fluid"; WO2017/202932 and WO2017/182599] or by other methods of exerting a force on particles in a microfluidic channel taken from the state of the art. As the distance from the electroporation zone to the separation zone is known and also the velocity of the cells as they move along the microfluidic channel is known, one can determine the time of travel from the electroporation zone to the separation zone, and apply the pulse of force knowingly to each targeted cell to send it into the secondary microfluidic channel depending on the results of the electroporation or on the characteristics of the cell. Thus, the methods of the invention may also be employed to separate viable cells from non-viable cells, and separate viable transfection-competent cells from other cells in a cell population, all of which can be carried out a single chip.

In one embodiment, the fluidic device forks into at least two fluidic channels at a forking point at or downstream of the cell separation module.

In any embodiment, the system comprises a cell transfection chamber fluidically connected to one of the fluidic channels downstream of the forking point.

In any embodiment, the cell electroporation module is configured to apply an electroporation voltage of 200-10,000 V/cm across the microfluidic channel. The magnitude of the voltage depends on the type of cells to be subjected to transfection, separation between the electrodes and some other characteristics such as pH of the cell carrying fluid and the guidance fluid and orientation of the cell with respect to the direction of the electric field and the current. According to one embodiment, the voltage applied is less than 1 V in amplitude, according to another embodiment, the applied voltage is in the range of 1-10 V.

According to another embodiment the applied voltage in the range of 10 V to 200 V. The polarity of the applied voltage could be positive or negative depending on the embodiment or could also be in the form of an AC voltage. Electric field created by the applied voltage depends on the separation between the electroporation electrodes or if one electroporation electrode is used, then the field depends on the separation between the electroporation electrodes and the electrodes connected to a fixed potential point. In some embodiments the electric field is less than 100 V/cm, in another embodiment it is less than 1000 V/cm and in another embodiment it is in excess of 5000 V/cm. In one embodiment the electric potential generated by the voltage/current source connected to the electroporation electrode is constant. In another embodiment the voltage generated by the voltage/current source has a form of a pulse. In one embodiment the duration of the pulse is less than 100 microseconds and in another embodiment the duration of the pulse is less than 1 millisecond, and in another embodiment the duration of the pulse is less than 10 milliseconds.

In any embodiment, the system comprises a shielding electrode module disposed adjacent the or each detection electrode module.

In any embodiment, the pump is configured to pump the population of cells and carrier fluid along the microfluidic channel with a linear flow velocity in the range of 0.05-2 m/s.

In any embodiment, the system is configured to continuously pump cells along the microfluidic channel at a rate of at least 50, 100, 250, 500, 750, 1000, 2000, 5000, or 20000 cells per second.

In any embodiment the electroporation module at least one pair of electroporation electrodes is connected to a voltage/current source capable of generating voltage/current pulse on demand between the electrodes. In one embodiment there is only one electroporation electrode in the electroporation zone and the electric field/current is established between this electrode and a ground electrode/electrodes. This is achieved by applying a voltage to the electroporation electrode. Such ground electrode/electrodes are present in a typical embodiment with the purpose of electrical separation of electroporation zone from other zones adjacent the electroporation zone and positioned along the microfluidic channel and capable of measuring AC electric characteristics of each single cell passing along the microfluidic channel. In other embodiments there are two electroporation electrodes in the electroporation zone positioned on two opposite sides of the microfluidic channel so that the electric field and the current produced by the electroporation electrodes is substantially misaligned with the flow direction in the microfluidic channel, e.g. the current and the electric field are directed nearly perpendicular to the flow direction.

In any embodiment the electroporation electrodes form an array of parallel conducting lines positioned on one side of the channel. These lines are of two subsets: subset A and subset B and they are positioned in alteration: line A- line B- line A- line B, etc. The lines A and B are connected to two different voltage points VA and VB. These could be DC voltages, AC voltages or voltage pulses. In this way, there is potential difference created between the lines A and B; and the pattern of electric field/current alternating in space is established. This pattern can be of static character in time or of alternating character in time. The lines could be aligned along the flow direction, or perpendicular to the flow direction, or being directed at some tilted angle with respect to the flow direction.

In any embodiment the electroporation electrodes are comprised of just two conducting stripes positioned on one wall of the channel and connected to two different points of electric potential.

In any embodiment the lines are positioned along two walls of the channel merging to form a corner of a microfluidic channel thus creating a region of a strong electric field proximal the corner region.

Preferably, the electroporation electrode/electrodes are positioned in electrical communication with the interior of the microfluidic channel. In one embodiment the electroporation electrodes are not positioned in electrical communication with the interior of the microfluidic channel but rather are separated by a thin layer of dielectric material from it. Such electrodes are connected to an AC voltage source for coupling of energy into the interior of the microfluidic channel via the capacitance formed by the dielectric layer.

In one embodiment the electroporation electrode/electrodes are formed on two flat substrates and these are then bonded together with the help of a polymer or a photoresist forming a channel of a rectangular cross-section so that two opposite walls are composed of two flat substrates having electrodes deposited on them and facing into the interior of the channel, and the other two opposite walls are formed of polymer/photoresist.

In another aspect, the invention provides a continuous high-throughput method of treating a population of cells as set out in Claim 15.

In any embodiment, the detection electrode module comprises a first detection electrode pair configured to detect a first change in electrical impedance across the microfluidic channel corresponding to the cell passing the first detection electrode pair and a second detection electrode pair configured to detect a second change in electrical impedance across the microfluidic channel corresponding to the cell passing the second detection electrode pair, wherein the method comprises analysing the detected first and second changes in electrical impedance to determine the time period T1 it takes for the cell to pass between the first and second detection electrode pairs, and calculating the velocity V of the cell based on the time period T1 and the distance D1 between the first and second detection electrode pairs.

In any embodiment, the method includes a step of transiently actuating the cell treatment module for a treatment period T2 corresponding to the cell passing all or part of the cell treatment module.

In any embodiment, the cell treatment module comprises a cell electroporation module and in which the method comprises the steps of electroporating the cell passing the cell electroporation module by passing a cell electroporation voltage across the microfluidic channel. In any embodiment, the carrier fluid may comprise a transfection reagent (i.e. "foreign material") and the method is a method of transfection of a population of cells or selected cells in a population of cells. In another embodiment, the method provides a population of electroporated cells, and the method may include a subsequent step of transfection of the electroporated cells. In one embodiment, the electroporated cells are viable and transfection competent cells.

In any embodiment, the method includes the steps of comparing the change in electrical impedance detected by the detection electrode module corresponding to a cell passing the upstream detection zone module with a reference change in electrical impedance, calculating a cell electroporation parameter selected from cell electroporation voltage or duration or number of cell electroporation voltage pulse(s) based on the comparison, and actuating the cell electroporation module to apply the electroporation parameter to the cell.

In any embodiment, the method comprises a step of hydrodynamically focussing the population of cells to provide a focussed stream of cell containing fluid upstream of the upstream detection zone. The focussed stream generally comprises a core cell-containing carrier fluid stream and a positioning stream of fluid forming a sheath around the core stream upstream of the electroporation zone.

In any embodiment, the hydrodynamic focussing step is configured to focus the cells asymmetrically in the microfluidic channel so that the train of cells are disposed closer to one electrode of a detection electrode module than a second electrode.

In any embodiment, the method includes the steps of detecting a downstream change in electrical impedance across the microfluidic channel at a downstream detection zone corresponding to a cell passing the downstream detection zone, comparing the downstream change in electrical impedance with a reference change in electrical impedance corresponding to a known cell electroporation status, and determining electroporation status of the cell based on the comparison.

In any embodiment, the comparison step comprises comparing the downstream change in electrical impedance corresponding to a cell passing the downstream detection zone with an upstream change in electrical impedance detected by the upstream detection zone corresponding to the same cell passing the upstream detection zone, and determine electroporation status of the cell based on the comparison.

In any embodiment, the electroporation status is selected from cell viability, successful cell electroporation, and unsuccessful cell electroporation.

In some embodiments, there are included steps of detecting a downstream change in electrical impedance across the microfluidic channel at a downstream detection zone corresponding to a cell passing the downstream detection zone, comparing the downstream change in electrical impedance with a reference change in electrical impedance corresponding to a known cell electroporation status, and actuating the cell electroporation module to modify the cell electroporation voltage or cell electroporation voltage pulse applied across the microfluidic channel based on the comparison.

In any embodiment, the comparison step comprises comparing the downstream change in electrical impedance corresponding to a cell passing the downstream detection zone with an upstream change in electrical impedance detected by the upstream detection zone corresponding to the same cell passing the upstream detection zone, and actuating the cell electroporation module to modify the cell electroporation voltage or the cell electroporation voltage pulse applied across the microfluidic channel based on the comparison.

In any embodiment, the fluidic device comprises a cell separation module downstream of the downstream detection zone, wherein the method comprises the step of actuating the cell separation module to separate selected cells from the population of cells based on the electroporation status of the cell.

In any embodiment, the separation step comprises calculating a time T2 it takes for a cell to travel from the one of the detection electrode modules to the cell separation module based on the determined velocity V and a distance D2 between the said detection electrode module and the cell separation module, and actuating the separation module a time T2 after the cell passes the said detection electrode module.

In any embodiment, the fluidic device forks into at least two fluidic channels at a forking point at or downstream of the cell separation module, wherein the method includes a step of selectively separating a cell or cells identified as having a common electroporation status into one of the fluidic channels.

In any embodiment, the method includes a step of the cell electroporation module transiently applying an electroporation voltage of 200-10000 V/cm across the microfluidic channel.

In any embodiment, the population of cells and carrier fluid is pumped along the microfluidic channel with a linear flow velocity in the range of 0.05-2 m/s.

In any embodiment, the population of cells and carrier fluid is pumped along the microfluidic channel at a rate of at least 500 cells/second.

In any embodiment, the carrier liquid comprises a cell transfection reagent.

Other aspects and preferred embodiments of the invention are defined and described in the other claims set out below.

### Brief Description of the Figures

**Figure 1****:** top view photograph of a chip with electrodes.
**Figure. 2a****:** Embodiment of a device containing electroporation zone with electroporation electrodes and three channels merging to achieve guidance of the cell-containing fluid by the sheath fluid.
**Figure 2b****.** Cross-section through the line A-A' of the channels of Fig. 2a.
**Figure. 3a****.** Embodiment containing electroporation zone and the alignment zone.
**Figure. 3b****:** cross-section of a channel with the cell-containing fluid and the shear fluid through the optional alignment electrodes.
**Figure. 4****:** top view of the microfluidic channel with electroporation electrodes comprising electroporation zone and primary detection zone.
**Figure. 5****:** schematics of the signals measured at the detection electrodes.
**Figure. 6a** cross-section through the microfluidic channel and the flow of the cell-containing fluid proximal one of the walls containing electroporation electrodes.
**Figure. 6b** cross-section through the microfluidic channel and the flow of the cell-containing fluid proximal one of the walls containing electroporation electrodes.
**Figure. 7****.** Cross-section through embodiment of microfluidic channel containing array of electroporation electrodes formed on one wall of the channel. The flow of cells is guided proximal the wall containing electroporation electrodes.
**Figure. 8a****.** Embodiment of containing one electroporation electrode formed on one wall of the channel.
**Figure. 8b****.** Embodiment of containing one electroporation electrode formed on two opposite walls of the channel.
**Figure. 9****.** Embodiment containing electroporation zone, secondary detection zone and the separation zone.
**Figure. 10****.** Embodiment with separation of cells ex-stiu of the microfluidic channel into cell-containing droplets.
**Figure 11****.** Photograph of the device shown in Fig. 1 performing sorting of droplets with electric filed. Three consecutive droplets removed in a periodic manner from a continuous train of droplets into a different target destination.
**Figure. 12a****.** Photograph of the device performing generation of smaller droplets at the rate of 33000 droplets per second.
**Fig. 12b****.** Photograph of the device performing generation of larger droplets at the rate of 27000 droplets per second.

### Detailed Description of the Invention

### Definitions and general preferences

Where used herein and unless specifically indicated otherwise, the following terms are intended to have the following meanings in addition to any broader (or narrower) meanings the terms might enjoy in the art:
Unless otherwise required by context, the use herein of the singular is to be read to include the plural and *vice versa.* The term "a" or "an" used in relation to an entity is to be read to refer to one or more of that entity. As such, the terms "a" (or "an"), "one or more," and "at least one" are used interchangeably herein.

As used herein, the term "comprise," or variations thereof such as "comprises" or "comprising," are to be read to indicate the inclusion of any recited integer (e.g. a feature, element, characteristic, property, method/process step or limitation) or group of integers (e.g. features, element, characteristics, properties, method/process steps or limitations) but not the exclusion of any other integer or group of integers. Thus, as used herein the term "comprising" is inclusive or open-ended and does not exclude additional, unrecited integers or method/process steps.

As used herein, the term "continuous" as applied to the method and system of the invention means that the cells are detected and electroporated as they are travelling along the microfluidic channel, i.e. it is not a static detection or electroporation, and does not involve the cells being shuttled backwards and forwards like in the prior art.

As used herein, the term "high-throughput as applied to the systems and methods of the invention means that the cells are pumped continuously along the microfluidic channel at a rate of at least 100 cells per second.

As used herein, the term "treatment of cells" refers to a physical or electrical manipulation of a cell. The invention is primarily related to electroporation of cells, or transfection of cells including electroporation, but it may also apply to methods and systems for other types of cell treatment, for example cell sorting, cell charging, cell deflection where the time of arrival of a cell at the treatment module, and the transit time of a cell past the treatment module, are important considerations.

As used herein, the term "transfection" means the process by which foreign material generally nucleic acid material, is introduced into the cell. As described above, various methods are described in the literature for transfecting cells, including methods that involve physical disruption of the cell membrane to allow introduction of foreign material into the cell through the disrupted cell membrane. The methods of the invention comprise electrical disruption of cell membranes to allow transfection (i.e. electroporation), and generally exclude virus-mediated cell transfection (transduction). The term includes stable and transient transfection, and transfection with DNA and RNA.

As used herein, the term "foreign material" refers to the material that is introduced into the cell during the process of disruption. The foreign material is generally nucleic acid material, for example DNA or RNA, which may be naked or purified, or form part of a nucleic acid construct including a transgene and other functional components such as promotors, poly adenylation tails and Kozak sequences (cloning vectors, plasmids, expression vectors, and artificial chromosomes).

As used herein the term "Cells" means any type of cell, including mammalian and non-mammalian cells such as white blood cells, red blood cells, bone marrow cells, immune cells, epithelial cells, nerve cells, pulmonary cells, vascular cells, hepatic cells, other non-hepatic liver forming cells, kidney cells, skin cells, stem cells, or bacterial and fungal cells and hybridomas, plant cells, protoplasts, pollen cells. The device and methods of the invention may be employed to prepare cells for transfection, and optionally to monitor the cells to detect and/or separate transfection competent cells. The device and methods of the invention may be employed to transfect cells, and optionally to monitor the cells to detect and/or separate transfected cells. The device and methods of the invention may be employed to prepare cells for transfection or transfect cells, and optionally to monitor the cells for viable cells and optionally separate viable and non-viable cells.

As used herein, the term "Focussed stream of cell-containing fluid" means a fluid containing cells in the form of a core stream containing the cells and a positioning stream that at least partially, or possibly fully, embraces/envelopes the core stream. We imply that the terms "cell-containing fluid", "cell-containing carrier fluid", "cell-containing liquid" and "cell-containing carrier liquid" have the same meaning in this specification. In one embodiment the cells in the core stream of the cell-containing carrier fluid are focussed into a single file arrangement. In one embodiment, the cells in the stream of the cell-containing fluid are aligned in the same direction. For example, if the cells are not spherical as is often the case but disk-shape or ellipsoid-shape, the cells in the set are aligned with the long axis of the ellipsoid or short axis of the disk, all in the same direction. In one embodiment the core stream is positioned between the positioning stream and at least one wall of the channel. Methods and devices configured for hydrodynamic focussing cells in a liquid stream are described in WO2017/182599 or EP 16166177.2-1371 "A microfluidic chip for focussing a stream of particulate containing fluid" as well as in other publications on microfluidics.

As used herein, the term "Microfluidic channel" means a channel having a cross-sectional area of less than 4 mm squared and a length of at least 1mm. In one embodiment, the microfluidic channel has a cross-sectional area of less than 0.25 mm squared. In one embodiment, the microfluidic channel has a cross-sectional area of less than 0.01 mmsquared. In one embodiment, the microfluidic channel has a cross-sectional area of less than 0.0025 mm squared. In one embodiment, the microfluidic channel has a length of at least 50 mm. In one embodiment, the microfluidic channel has a length of at least 200 mm. Generally, the microfluidic channel is provided on a substrate such as a chip. In one embodiment, the microfluidic chip comprises a plurality of layers, for example at least 2, 3, 4, 5, 6, 7, 8, 9 or 10 layers. In one embodiment, the cross-sectional area of the microfluidic channel is constant along its length. In one embodiment, the cross-sectional area of the microfluidic channel is variable along its length. In one embodiment, the cross-sectional area of the microfluidic channel downstream of the electroporation zone is smaller than the cross-sectional area upstream of the electroporation zone. In one embodiment, the cross-sectional area of the microfluidic channel upstream of the detection zone is smaller than the cross-sectional area downstream of the detection zone.

As used herein, the term "Detection zone" means a section of the microfluidic channel at which a sensor such as an electrode pair is located. Typically, the sensor includes at least one excitation electrode and at least two sensing electrodes. The sensor may be configured to detect AC impedance changes in the microfluidic channel caused by single cells passing through the sensor, i.e. changes in impedance detected at the detection electrode. The changes may include changes in amplitude, phase, or amplitude and phase of the signal. The sensor may also include shielding electrodes usually connected to a fixed potential point and positioned at the periphery of the detection zone. The shielding electrodes are used to reduce the noise and spurious signal in the sensor. Details of such sensors are described in the patent applications WO2017/202932 and WO2017/182599. In one embodiment there is only one detection zone. If the detection zone is located upstream of the detection zone we refer to it as a primary electroporation zone. Where it is located downstream from the detection zone, we refer to it as a secondary electroporation zone even if there is only one detection zone. In some embodiments there are two detection zones. We shall follow the same convention in the terminology. The detection zone located upstream of the electroporation zone is called a primary detection zone and the one located downstream of the electroporation zone, is called a secondary electroporation zone. In some embodiments there are three or more detection zones. In one embodiment some of the electrodes of the detection zone are also combined with the electrodes of the electroporation zone. This is achieved by using the methods of frequency separation. For example, the electroporation electrode may be energised by a DC voltage of a constant magnitude or by a pulse of DC voltage. At the same time this electrode can be connected to an input of a pre-amplifier of a lock-in amplifier via a high-pass or band-pass filter. By using analogue and digital filters and the methods of frequency separation, one can separate the electroporation voltage from the voltage of the signal induced from cells passing though the detection zone. We describe the concept of frequency separation in the detection zone in EP 17177631.3-1553, "Apparatus and Method for Improved Identification of Particles and Cells".

As used herein, the term "Separation zone" is a part of the microfluidic channel, distal of the treatment (i.e. electroporation) zone, where cells in the fluid can be separated based on the parameter changes in the channel detected at the detection zone(s). The separation zone generally includes a force generator operably connected to the detection sensor and configured to exert a force on the cells in response to signals from the detection zone, to separate the one or more particulates from the stream of fluid. Examples of suitable force generators for use in cell sorting apparatus are well known in the art and described for example in Wyatt Shields et al ("Microfluidic Cell Sorting: A Review of the Advances in the Separation of Cells from Debulking to Rare Cell Isolation", Shields C.W. et al, Lab Chip. 2015 February 16, 15(5): 1230-1249). In one embodiment, the device will typically include a processor operably connected to the at least one sensor and the force generator and configured to actuate the force generator in response to a signal received from the sensor. The actuating signal may be pre-programmed into the processor and may vary from cell type to cell type. The separation force could be also a result asymmetric pattern of electric current and electric fields created at the split of a microfluidic channel into two or more secondary channels. This is described in detail in the patent application [EP 17177624.8-1553, "A microfluidic apparatus for separation of particulates in a fluid"] incorporated here as prior art.

The term "Electroporation Zone" as used herein is a part of the microfluidic channel equipped with a number of electroporation electrodes. Usually the electroporation electrodes are positioned on the opposite sides of the channel. In one embodiment there are two electroporation electrodes on the opposite sides of the channel facing each other and making electric contact with the interior part of the microfluidic channel. The electrodes are electrically connected to a voltage source. In such an embodiment, when the two electroporation electrodes are connected to a voltage source, there is electric field generated in the direction substantially perpendicular to the direction of the flow of liquid in the channel. In one embodiment the electric field is of the value in the range 50-100 V/cm, in another embodiment the field is in the range of 100-5000 V/cm, in another embodiment the field is in excess of 5000 V/cm. In some embodiments the Voltage Source could generate a fixed value of DC voltage, for example, 1V or 5 V or 100 V, depending on the separation between the opposite electroporation electrodes. In other embodiments the Voltage Source could generate an AC voltage of a fixed amplitude. The amplitude could be above 1 V, or above 5 V or above 100 V depending on the embodiment. The frequency of the AC voltage could be significantly higher than the inverse of the time required for the cells to pass through the electroporation zone. For example if the length of the Electroporation Zone along the channel is 50 microns and the linear velocity of the cells in the microfluidic channel is 50 mm/sec, this means the time required for the cells to pass though the electroporation zone is 1mS and the AC frequency (if the specific embodiment does use AC voltage as opposed to DC voltage) is then significantly higher than 1 kHz, e.g. equal to 5 kHz or 10 kHz or 30 kHz. In some embodiments the voltage source could be a generator capable of generating voltage pulses/current pulses on demand. The amplitude, timing and the duration of the pulse is controlled by the voltage source controller. The details of these are given in the description of the embodiments. We describe the source connected to the electroporation electrodes as the voltage source (DC, AC or voltage on demand). However, it should be noticed that in generating the voltage, the voltage source also generates the current. Part of the current is to charge the capacitive or inductive component of the electroporation electrodes and the associated circuitry and another part of the current is due to a finite resistance/impedance between the electroporation electrodes. Nonetheless we shall refer to the source connected to the electrodes as the voltage source even though it is understood by those skilled in the art that it is also a current source.

As used herein, the term "electroporation parameter" refers generally to the amplitude of the voltage applied by the electroporation module, the duration of the electroporation voltage pulse, or the number of electroporation voltage pulses.

### Exemplification

The invention will now be described with reference to specific Examples. These are merely exemplary and for illustrative purposes only: they are not intended to be limiting in any way to the scope of the monopoly claimed or to the invention described. These examples constitute the best mode currently contemplated for practicing the invention.

To best understand the invention, one needs to consider a flow of cells suspended in a cell-containing carrier fluid carrying the cells in a microfluidic channel. In the present case the microfluidic channel has a cross-section that is broadly comparable with the size of the cells, and yet larger than the size of the cell, e.g. 1 to 1000 times larger than the size of the cell. These figures are given here as a rough indication, and the dimensions of the channel outside this range can be also deployed; for example, the channel could be 2000 times larger than the size of the cells. The microfluidic channel may also be referred to as the channel for shortness. The mass density of the cells (i.e. mass of the cell divided by the volume of the cell) is not exactly equal to the mass density of the carrying liquid, even though these two densities are normally broadly comparable. The liquid carrying cells could be any physiological saline buffer e.g. PBS (phosphate saline buffer), TRIS buffered saline, DMEM (Dulbecco's Modified Eagle's Medium). Various other liquids commonly used in cell laboratory, pharmaceutical and medical work could also be deployed as the cell carrying liquid. For the electroporation the cells are suspended in electroporation buffer. Compositions of the electroporation buffers are well known to those practicing electroporation. This is description of one specific electroporation buffer. The buffer is composed of 8 mM Na2HPO4, 2 mM KH2PO4, 1 mM MgSO4·7H2O and 250 mM sucrose, pH 7.4. To make the buffer one could dissolve 1.136 g of Na2H·PO4, 0.272 g of KH2PO4, 0.2465 g of MgSO4·7H2O and 85.575 g of sucrose in 1 litre of water, and adjust pH to 7.4. The buffer solution is then filtered with a 0.2-µm membrane and stored at 4 °C. Another example of a suitable buffer solution is as follows: 13 mM KH2PO4, 13 mM K2HPO4 and 25 mM myo-Inositol, pH 7.2. Buffers containing PO4 complexes are known to ensure better cell viability during electroporation than KCI-based buffers. The difference in densities of the cells and of the liquid is defined by the type of the cells and the type of the cell-carrying liquid.

To verify electroporation one can use fluorescently labelled molecules in the buffer such as fluorescently labelled DNA, silencing siRNA or dextran and trypan blue. The DNA concentration of 100-2000 µg ml-1 is used.

The fluorescence is detected using Zeiss AxioVert A1-FL-LED microscope equipped with fluorescence filter set for FITC fluorescence measurement, for example filter set 09 (Excitation BP 450-490, Bandstop FT 510, Emission LP 515).

Fig 1 (photograph of a channel shows microfluidic channel of a nearly rectangular cross-section. There are gold electrodes with the thickness of 200 nm to 1 um marked by the numeral 1. The rectangular cross-section is not meant to be the only option, and channels with other cross-sections are also possible. The dimensions of the channel's cross-section are 30 micron by 30 micron in the detection zone. The channel is made out of SU-8 photoresist which is deposited onto acrylic or glass substrates using conventional photolithography.

The cells are aligned in a train so that one cell passes through any given cross-section perpendicular to the flow, at a time. Examples of cells that can be used in these experiments include HEK-293A, CHO-K1, yeast (Saccharomyces cerevisiae), sperm cells and HeLa cells. These are only given here as examples, and numerous other cells are possible. The rate of cells passing through the channel is up to 50,000 cells per second. The rate of the cells flowing is controlled by the flow velocity in the channel and the concentration of cell in the cell-containing carrier fluid. The concentration of cells is in the range of 1 × 10⁵ - 5 × 10⁷ cells per ml in the electroporation buffer. The concentration of cells is confirmed using a flow cytometer Accuri C6 Plus, BD Biosciences. The linear flow velocity in the channel is in the range of 0.01m-5 m/s. At this point we should clarify the point of the linear velocity of the cells in the channel. If the flow of the liquid in a channel is driven by a pressure differential along the channel, the linear velocity of the liquid will vary across the channel. The velocity is normally highest at the central area of the channel cross-section and lowest along the walls of the channel, although the details of the velocity differences are determined by the geometry of the cross-section of the channel. If the flow is sustained e.g. by electroosmotic forces as opposed to a mechanical pressure differential applied along the channel, the distribution of the flow across the channel may be different but nonetheless, usually the linear velocity of the flow varies across different points in the channel's cross-section. This may result in the difference in the flow velocities along different sides of a cell: fluid around the part of the cell closest to the wall moves slower than fluid around the part of the wall closest to the centre of the channel. Consequently, this may result in a hydrodynamic force acting on the cell having a component perpendicular to axis of the channel. The value of the hydrodynamic force acting on the cell in the flow of the cell-carrying fluid is determined by the flow of the fluid in the channel and the cell's position within the channel. The combination of the gravity force, buoyancy of the fluid and the hydrodynamic force may result in preferential positioning of the cell at some parts of the cross-section of the channel, resulting in aggregation of the cells e.g. mainly towards the centre or mainly towards the lower (floor) side of the channel or towards the upper (ceiling) side of the channel. The preferential positions of the cells are determined by the shape of the channel, hydrodynamic characteristics of the cells ( mass density of the cell, shape of the cell) and density of the cell-carrying liquid and also by the orientation of the channel with respect to the gravity direction.

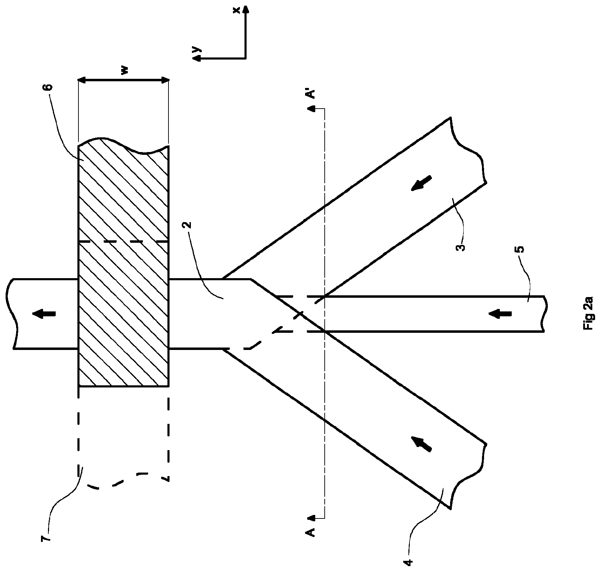

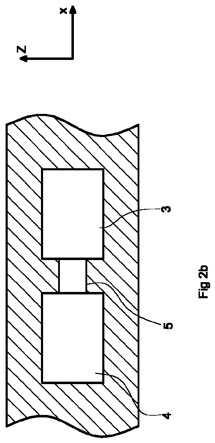

Figs. 2a, 2b show schematics of an embodiment of the invention. There is a common microfluidic channel marked by the numeral 2 (also referred to as a microfluidic channel), the said channel is of a rectangular cross-section with a width of 100 microns and a height of 100 microns. The common microfluidic channel 2 is formed by merging three microfluidic channels together. The three channels are the guidance channels one and two marked by the numerals 3 and 4 and the sample microfluidic channel 5. The guidance channels one 3 and two 4 are supplied with sheath fluid, and the sample microfluidic channel 5 is supplied with the cell-containing carrier fluid. The channels are connected to pressure controlled pumps such as e.g. UNIGO or 4U from Cellix Ltd. Pumps from other vendors could also be deployed. The typical flow in the channels is in the range of 1-2000 µl/min but flow rates outside this range are also possible. In a typical embodiment the channels are connected to the pumps at the upstream end and there is no pump connected to the common microfluidic channel 2 at the downstream end. However, in some embodiments there is also a pressure source (pump) connected to the common microfluidic channel 2 downstream of the electroporation zone. The pumps are not shown in Fig. 2a and indeed all other figures of this document. The figures 2a, 3a, 4, 7, 8, 9 of this document do not show the entire microfluidic chip. They only show a section of the microfluidic channel or an array of channels with different zones, and electrodes positioned along the channels. It should be appreciated by those skilled in the art that microfluidic chip contains other means such as means for connection of the channel to the pumping means, wells or reservoirs for collecting the liquid at the exit from the channel and other means commonly used in microfluidics. We do not show them in the figures and do not discuss them for brevity. They are assumed to be present in each embodiment as required. There are also means for flow control so that the desired flow of the liquid can be independently established and controlled in each channel. Normally these means for the flow control are integrated with the pumps, as is the case in EXIGO, UNIGO or 4U from Cellix Ltd, but in other pumps they could operate as separate devices, physically detached from the pump and operating/communicating with the pump by electronic means to provide feedback for the flow control. The flow control means are also not shown in figures of this specification and they are assumed to be present in the device and method described.

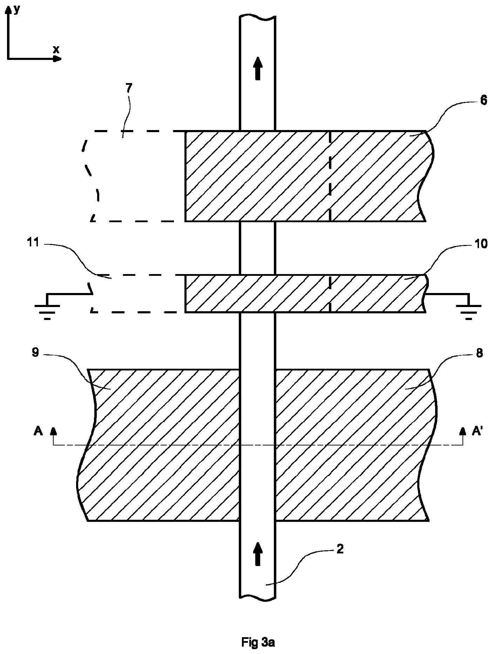

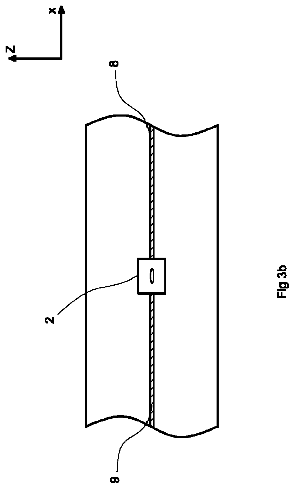

The flow direction is indicated, and it is directed from the three merging channels into the common microfluidic channel 2. In this way the hydrodynamic focussing localises the flow of the cell-containing carrier fluid within the cross-section of the common microfluidic channel 2, and the position of the cell-containing carrier fluid is defined by the flows and the pressure values applied at the guidance channel one 3, guidance channel two 4 and the sample microfluidic channel 5. For example, if the pressure in the guidance channel one 3 is increased while the pressure in the guidance channel two 4 stays constant, the flow of the cell-carrying fluid displaces leftwards within the common microfluidic channel 2 with reference to Fig. 2a. This is well appreciated by those skilled in the art of microfluidics and described in detail in the patent application EP 17177619.8-1553 "A microfluidic chip". We can also control width of the cell-carrying fluid flow by varying the ratios between the flows in the three channels: the guidance channels one 3 and two 4 and also the sample microfluidic channel 5. According to the invention, the flow of cell-carrying fluid is set so that the cells travel in a single file, one cell after another moving along the same lime in through the channel, preferably with the separation between the cells being greater than the average size of the cells. In one embodiment the separation between the cells is a fraction of the size of the cells, e.g. approximately half of the size of the cells. In another embodiment, the separation is 1-10 times greater than the size of the cells. In another embodiment the separation is some 10-100 times greater than the size of the cells. In other embodiments, it is over 100 times greater. The separation between the cells can be controlled by changing the concentration of the cells in the buffer solution. There is further the electroporation zone composed of two electrodes 6,7 aligned perpendicular to the channel direction: one at the top wall of the channel and another one at the bottom wall, the width w of the electrodes is 200 microns. In Fig. 2a, the two electroporation electrodes 6,7 are not distinguished as the figure shows the top view. Electroporation electrodes typically have a width w in the range of 5 micron to 5 mm. The electroporation electrodes 6,7 are in electrical communication with the interior of the microfluidic channel 2 and they are connected to a voltage source. In one embodiment the voltage source is a DC source, capable of generating the value of the voltage up to 40 V. In another embodiment the electroporation electrodes 6,7 are connected to an AC voltage source with the frequency of up to 200 kHz and the amplitude of up to 50 V. In another embodiment the voltage source is a pulse generator capable of generating voltage pulses of the height of 50 V and pulse duration of 10⁻⁵- 10⁻² s. According to one embodiment, the DC or AC voltage is applied to the electroporation electrodes 6,7 generating a vertical electric field between the electroporation electrodes. Figs 2b shows a cross-section through the line AA' of Fig. 2a. According to one embodiment the cells are positioned in the middle of the common microfluidic channel 2 and they are not aligned in any particular way with respect to the electroporation electrodes 6,7. According to another embodiment, all the cells in the train of cells are aligned in the same way with respect to the electroporation electrodes 6,7. This can be achieved e.g. using the method of anisotropic hydrodynamic focussing as described in European Patent Application No 16166177.2-1371 "A microfluidic chip for focussing a stream of particulate containing fluid" (see Figs. 18a, 18b, 19a, 19b, 20a, 20b) or by using alignment with the help of an electric field as described in the patent application EP 17177619.8-1553 "A microfluidic chip". Alignment with the help of an electric field normally requires the presence of further electrodes, the alignment electrodes. These are shown in Fig. 3a, 3b and are marked by the numerals 8,9. The alignment electrodes 8,9 positioned along the microfluidic channel 2 form an alignment zone typically located upstream from the electroporation zone. The alignment electrodes 8,9 are connected to a DC or an AC voltage source to generate the alignment electric field. Fig. 3a and 3b shows two alignment electrodes 8,9 connected by lines 1 and 2 to the alignment voltage source (not shown in Figs. 3a and 3b). There are two shielding electrodes 10,11, one at the top wall of the microfluidic channel 2 and one at the bottom wall, positioned between the electroporation electrodes 6,7 and the alignment electrodes 8,9. The shielding electrodes 10,11 are connected to a ground potential or another fixed voltage and serve the purpose of separating the electroporation zone from the alignment zone. The alignment voltage source generates an alignment electric field between the two alignment electrodes 8,9. This alignment electric field penetrates through the train of cells rotating the cells if they have anisotropic shape. This is schematically shown in Fig. 3b depicting a cross-section through the alignment electrodes 8,9, along the line AA' of Fig. 3a containing a disk-shape cell aligned with the flatter surface parallel to the direction of the electric field. In this case the alignment electric field is directed mainly along the X axis and perpendicular to the electroporation field. Other embodiments are also possible. Alignment with the help of anisotropic hydrodynamic focussing is another possibility for aligning cells and would normally require positioning the cell-containing carrier fluid not in the centre of the microfluidic channel 2 but rather in proximity of one of the walls or one of the corners of the microfluidic channel 2 as described in Patent Applications WO2017/182599 EP 16166177.2-1371 "A microfluidic chip for focussing a stream of particulate containing fluid". According to the invention, such a uniform positioning of the cells with respect to the direction of the electric field offers better uniformity of the electroporation conditions and this translates into better transfection-competent electroporation and better viability of the cells after the electroporation.

All the figures of this document also do not show a fluorescence microscope such as Zeiss Axiovert A1-FL-LED microscope even though some embodiments described here are expected to operate with the assistance of such a microscope.

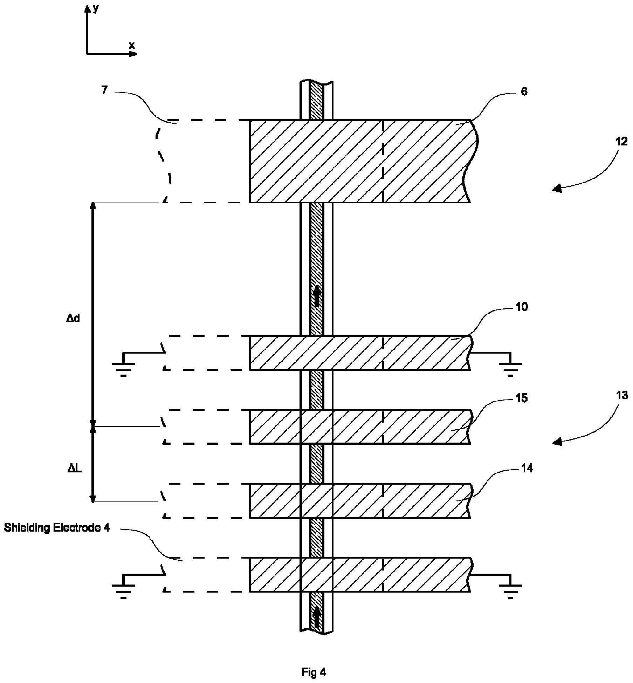

Fig. 4 shows an embodiment of the device comprising an electroporation zone 12 and a primary detection zone 13, located upstream from the electroporation zone 12. There could be also optional alignment zone, not shown in Fig. 4. Such alignment zone is preferably located upstream from the detection zone 13. The detection zone 13 comprises a sensor capable of detecting changes in the AC impedance measured between electrodes across the microfluidic channel 2 caused by the passing cells. Operation of the sensor is described in detail in the patent applications
[EP 17177631.3-1553, "Apparatus and Method for Improved Identification of Particles and Cells";
EP 17177662.8-1553, "System and Method for Improved Identification of Particles or Cells"] which are referred here as prior art. We only outline the operation of the sensor briefly. There are typically at least two detection electrodes 14,15 connected to a pre-amplifier, a comparator comparing the signals from the two detection electrodes 14,15 , and a digital lock-in amplifier. The said two detection electrodes are shown in Fig. 4. There is also at least one excitation electrode connected to an AC generator. The excitation electrode is typically located on the opposite wall of the microfluidic channel and is not shown in Fig. 4. There are also two shielding electrodes 10,11, one of these is located on the upper wall of the channel and the other one at the lower wall. The shielding electrodes 10,11 are connected to a point of fixed potential such as ground potential and serve the purpose of separating electrodes of the sensor from the spurious and parasitic signals originating in other parts of the microfluidic channel. The separation between the detection zone 13 and the electroporation zone 12 is typically in the range of 0.1mm to 100 mm and more typically in the range of 1mm to 20 mm.

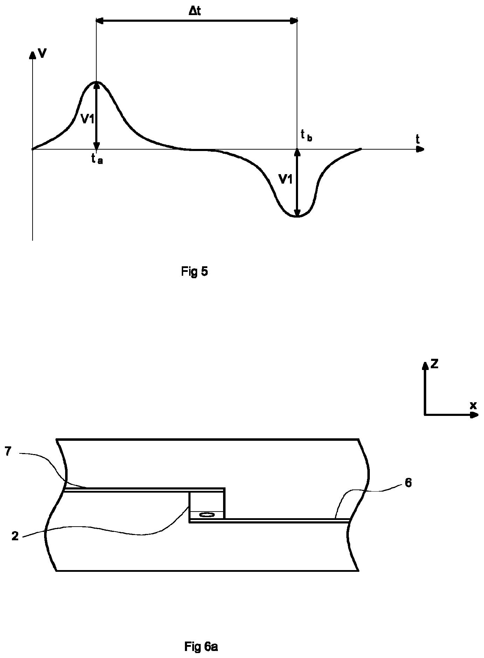

The key point is the coherent operation of the electroporation zone 12 and the detection zone 13. Each cell passing through the detection zone 12 is detected by the cell sensor and the moment of the cell's passing through the first and second detection electrodes 14,15 is detected. The velocity of the cells in the train of cells can be calculated from the shape of the signal detected at the two detection electrodes 14,15. The typical shape of the signal is shown in Fig. 5. There are two peaks corresponding to the cell passing proximal the first and the second detection electrodes 14,15. These are typically of similar or near-similar amplitudes and having opposite polarities as the two detection electrodes 14,15 are connected to the inverted and non-inverted inputs of a comparator. The time interval between the inverted and the non-inverted peaks Δt, is equal to the distance between the electrodes multiplied by the cell velocity. There is some ambiguity on what should be regarded is the distance between the electrodes. As a starting point one can take this as the distance between the centre lines of the first and the second detection electrodes marked by numerals 14,15. This distance is marked as Δl in Fig. 4. However, a more accurate analysis suggests that this distance depends on the configuration of the excitation electrodes and also on the position of the stream of the sample fluid within the microfluidic channel 2, i.e. whether it passes through the centre of the channel, or closer to the excitation electrodes, or closer to the detection electrodes. It is desirable to calibrate this distance Δl by an independent measurement. For this, it is advisable to pass a train of cells at a known velocity. Such velocity could be independently measured by means of the optical microscope capturing the images of the cells and processing them to extract the cell velocity. The signals from individual passing cells are also detected in the detection zone 13 using the two detection electrodes 14,15 as described above. In this way one can multiply the time interval Δt shown in Fig. 5 by the velocity of the cells to obtain the effective value of Δl. This effective value can subsequently be used for the real monitoring of the cell velocity. It should be emphasised that as the effective length Δl is somewhat dependent on the position of the cells within the microfluidic channel 2, the position of the sample fluid within the channel during the calibration should be same as the intended position during the electroporation work described in the specification.

Once the velocity of the cells is known, and the distance from the detection zone 13 to the electroporation zone 12 is known, one can identify the time travelled by the cell from the centre of a detection electrode 14,15 (e.g. last detection electrode) to the electroporation zone 12. This distance is marked as Δd in Fig. 4. The moment of passing the second detection electrode is marked by tb in Fig. 5. The time of travel is equal to the ratio of the distance Δd by the velocity of the cells. We shall denote this time as tdelay. According to the invention one has to activate the voltage source connected to the electroporation electrodes 6,7 with the delay of t_{delay} after the cell passed the second electrode 15 of the detection zone 13. Therefore, the operation of the system is as follows. The signal from the cell sensor is detected and processed to identify parameters of the cell. Then depending on the protocol of the electroporation procedure, the electroporation electrodes 6,7 are activated with the known time-delay later. Examples of the protocol of the procedure could include:
i. perform electroporation on each passing cell,
   or
ii. perform electroporation on each cell whose electrical characteristics as measured by the sensor fall in a certain range (or several ranges) of values. For example, the set of cells could consist of two or more subsets characterised by different values of the electrical parameters as detected by sensor, and one may need to perform electroporation on just one subset.
   or
iii. do not perform electroporation on cells whose electrical characteristics as measured by the sensor fall in a certain range (multiple ranges) of values.
   or
iv. perform electroporation on a fraction of the cells that have (do not have) electrical characteristics in a certain range of values, for example, every second cell of a certain type is subjected to electroporation.

There could be other more complex protocols as defined by the requirements of the transfection procedure.

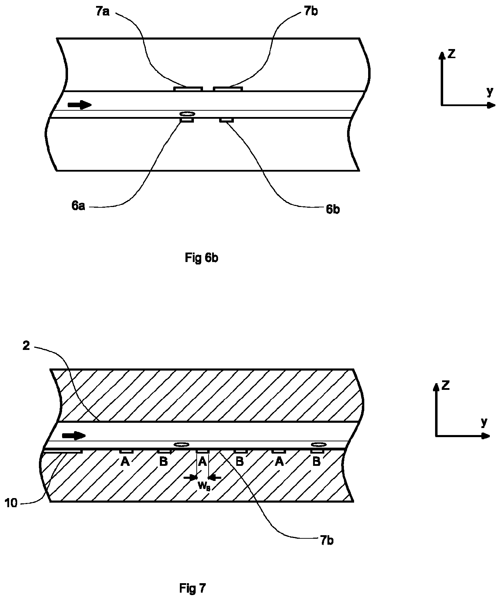

The sample fluid in the microfluidic channel 2 does not need to pass through the central part of the channel's cross-section. This is shown in Figs. 6a and 6b. In this embodiment the electroporation electrodes 6,7 on the lower wall of the microfluidic channel 2 are smaller in size than the ones at the upper wall. This means the field resulting from the voltage between the electrodes e.g. electrodes 6a and 7a, is stronger in the vicinity of the lower electrode 6a than in the middle of the channel or in the vicinity of the electrode 7a. The cells are forced to pass in the vicinity of the electrodes 6a and 6b as is shown schematically in Fig. 6b. This positioning of the sample carrying fluid in vicinity of one of the walls of the microfluidic channel is achieved by asymmetric merging of the sample microfluidic channel and the guidance channel so the that the sample microfluidic channel is positioned adjacent the wall where the electrodes 6a and 6b are located. In this embodiment the cells are shown to be aligned. The methods of cell alignment in the microfluidic channel 2 under the influence of hydrodynamic focussing and proximity to the channel wall, are described earlier. The advantage of aligning the anisotropic cells is that electroporation electric field is directed in the same direction with respect to all the cells thus leading to a reduced spread of values of the critical electroporation field that is cell-orientation dependent and better homogeneity of the electroporation result across a population of cells.

Fig. 7 shows an embodiment where electroporation electrodes 6,7 are positioned on one wall of the microfluidic channel 2. In this embodiment the electroporation electrodes 6,7 form an array of stripes aligned perpendicular to the flow direction and connected to two electric different potential points of the voltage source (AC, DC or pulse voltage generator). The width of the stripes is between 10 microns and 400 microns. This is marked as Ws in Fig. 7. The number of stripes in the array can typically be from 2 to over 50, but the number outside this range could also be used. In this embodiment the stripes forming the electroporation electrodes 6,7 are divided into two groups: electrodes marked with letters A and B respectively. The electrodes marked with letters A are grouped together and connected to the same electric potential, the same applied for the electrodes of the group B. The electric potential (AC, DC or pulsed) is applied between the electrodes of the two groups and therefore the electric field is applied between the adjacent A and B electrodes. In this embodiment the dominant direction of the electric field sensed by flowing cells is along the flow direction. This is in contrast with the previous embodiments where the electroporation electric field is predominantly aligned perpendicular to the flow direction. In this case it is advantageous to guide the cells close to the wall of the channel containing the electroporation electrodes A and B as this exposes the cells to stronger field. In this embodiment there is an optional shielding electrode. Fig. 7 shows an embodiment with the lines of the electrodes A and B being perpendicular to the flow direction. They are also of the same width. One could also construct the embodiment with these lines parallel to the flow direction. One could have lines of the same width of different widths. One could also construct embodiment with lines positioned on two adjacent walls of the microfluidic channel thus forming the region of strong electric field in proximity to the corner. For brevity we do not show these embodiments on separate figures.

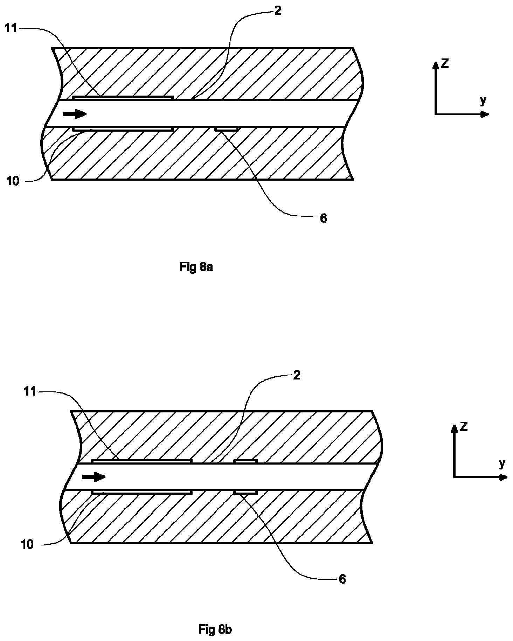

Figs. 8a and 8b show embodiments where there is only one electroporation electrode 6 and the electric field is generated between this electroporation electrode 6 and the shielding electrodes 10,11 separating the detection zone from the electroporation zone. The embodiment in Fig. 8a shows one electroporation electrode 6 positioned on one wall of the microfluidic channel 2. Fig. 8b shows the electroporation electrode 6 positioned on two opposite walls of the channel 2. It may appear that these are two separate electroporation electrodes but as they are connected to the same electric potential, we consider them as two areas of the same electroporation electrode 6 (electrode 6 and another unmarked electrode that is opposite to the electrode 6). The electric field is again created between the electroporation electrode/electrodes 6 and the shielding electrode 10,11. The shielding electrode 10,11 here can be either positioned downstream or upstream from the electroporation electrode 6 and for this reason we did not indicate the flow direction on the Figures 8a and 8b.

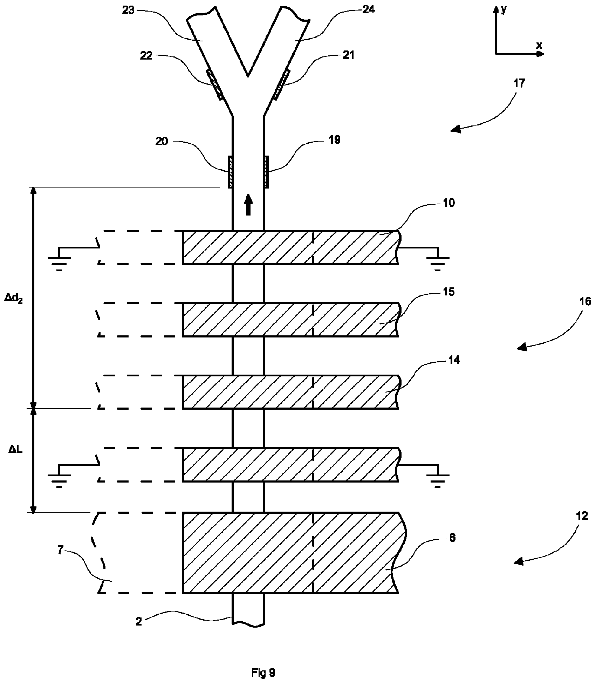

Fig. 9 shows an embodiment of the device comprising one electroporation zone 12, one secondary detection zone 16 and one separation zone 17. The secondary detection zone 16 is positioned downstream from the electroporation zone 12, and the separation zone 17 is positioned downstream from the secondary detection zone 16. The distances between the electrodes of the secondary detection zone 16 and the separation zone 17 labelled as Δd2, and between the secondary detection zone 16 and the electroporation zone ΔL are known. Since the linear velocity of the cells in the channel is known as described in relation to the earlier embodiments, the time of travel of each cell from the secondary detection zone 16 to the separation zone 17 is known. Likewise, the time of travel from the electroporation zone 16 to the secondary detection 16 zone is also known. It is desirable to calibrate for the time of travel, e.g. with independent optical detection of the cells as described in relation to some of the previous embodiments. Fig. 9 shows a top view of the channel and electrodes can normally be positioned under each other, we only show one of the two electroporation electrodes 6,7 and only two out of four detection electrodes 14,15. The operation of this embodiment is as follows: the electrodes in the electroporation zone 12 generate an electroporation field electroporating the cells. The voltage source connected to the electroporation electrodes 6,7, the first to fourth separation electrodes 19,20,21,22, and preamplifiers, comparator and locking-in amplifier-based electronics of the detection zone, are all not shown for brevity. They are the same or similar as described in relation to the some of the previous embodiments. The operation of the separation zone 17 is based on applying current/voltage pulse between the electrodes in asymmetric fashion. For example, for guidance of the cells from the common microfluidic channel 2 into a first separation channel 23, the potential is applied between the fourth and first separation electrodes 22,19, while the second and third separation electrodes 20,21 are at floating potential. For guidance of the cells from the common microfluidic channel 2 into a second separation channel 24, the same potential difference is now applied between the third and second separation electrodes 21,20 while the fourth and first separation electrodes 22 ,19 are now at floating potential. There are other options for the sequences of voltage pulses applied to the separation electrodes to achieve switching between a number of separation channels. This is described in EP 17177624.8-1553, "A microfluidic apparatus for separation of particulates in a fluid" what is incorporated here as state-of-the-art. The result of the electroporation is confirmed by the measurements of the electric characteristics of each passing cell in the secondary detection zone 16. If the electric characteristics of the cells as measured in the secondary detection zone 16 fall within the desired range of values, indicating e.g. that the membrane has been altered for the electroporation, then a known time delay later corresponding to the time of travel from the secondary electroporation zone to the separation zone, the electrodes is the separation zone are activated thus sending the cell to the desired destination channel (e.g. first separation channel 23), otherwise the electrodes in the separation zone are activated to send the cell into the second separation channel 24. In this embodiment cells could also be electroporated one cell at a time by applying pulses of voltage to the electroporation electrodes. For this, it is preferable to have the device equipped with the primary detection zone with the cell sensor as described in relation to Fig. 4. We will not present this embodiment in a separate figure for brevity but it should be appreciated that one can construct an embodiment with the one primary electroporation zone positioned upstream from the electroporation zone, and one secondary electroporation zone positioned downstream from the electroporation zone, a separation zone located downstream from the secondary detection zone. Indeed, one could add the further zones of the set described here, for example one could add a third detection zone downstream from the separation zone. The purpose of this would be to confirm that the cells have been separated according the range of the electrical parameters set up as a criterion for the cell separation. There could be a secondary separation zone where the cells in one separation channel are further separated into two or more subsets.

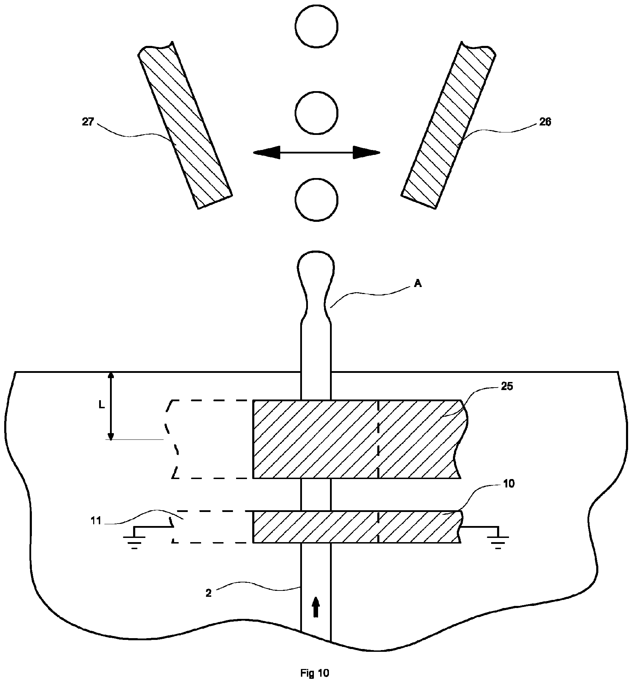

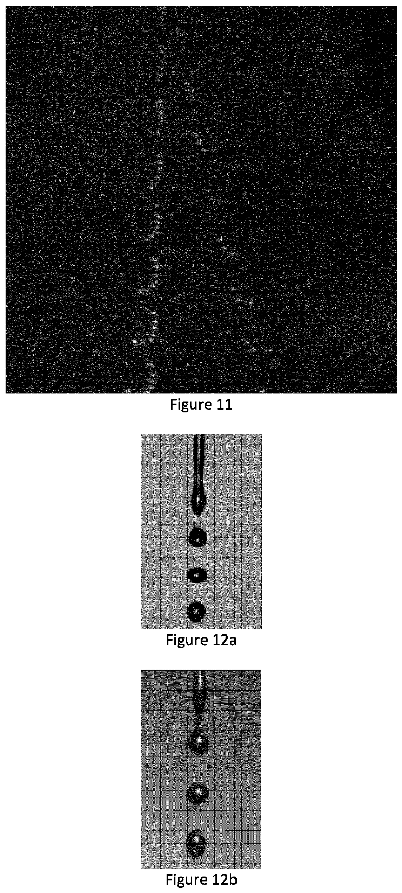

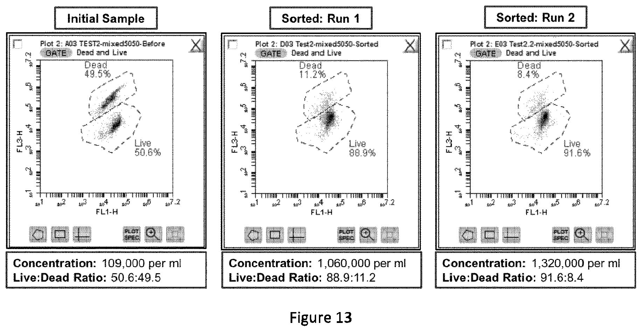

The cell separation zone 17 shown in Fig. 9 separating the cell on-chip, is only one embodiment out of several possible options. The cells could be also separated outside the microfluidic channel 2, i.e. ex-situ as shown schematically in Fig. 10. Photograph demonstrating operation of this method of cell separation is shown in Figs. 11 and Fig. 12a, Fig. 12b. This embodiment of the device fragments the jet of liquid emerging from the channel into a stream of highly regular droplets. Some of the droplets are charged by the charging electrode. The charging electrode (electrodes) is (are) located some L= 0-5 mm from either the shielding electrode (i.e. electrode connected to a fixed potential) of the detection zone or from electrodes of the electroporation zone. The charging electrode is labelled by the numeral 25 in Fig. 10. We found it beneficial to insert the shielding electrode in between the charging electrodes and other zones located upstream as described in some of the previous embodiments. The charging electrode in this embodiment is the last electrode along the microfluidic channel, located downstream from all other zones and close to the termination point of the microfluidic channel. The termination is an open end of the microfluidic channel exposing the channel to the ambient. The flow rate in this microfluidic channel is 0.1-5 m/s although the flow rates outside this range can also be deployed. The channel is of a rectangular cross-section although other cross-sections could also be deployed. The channel shown in Figs. 11, 12a, 12b is of square cross-section with the size of 30 microns. There is a piezo actuator mechanically coupled to the chip such as P-080 PICMA from PI (Germany). The piezoactuator is energised by a voltage source generating voltage pulses with the amplitude of up to 100 V. The polarity of the voltage depends on the specific type of the piezo element used. The charging electrode is energised with voltage pulses of the duration of 1-10 piezo periods and the amplitude of 1-10 V. The timing of the pulse is given by the electroporation and detection zone as described in relation to the previous embodiment. For this the distance from the electroporation zone or from the detection zone to the point where the jet ejected from the channel breaks into droplets, marked by the point A in Fig. 10, needs to be known. This point is located along the jet emerging from the microfluidic channel and outside the channel. The timing of pulse is calculated to ensure that the channel is energized at the time When the cell to be removed from the total set of cells reaches the point marked "Break". There are two guidance electrodes positioned just outside the exit from the channel. The length of these is in the range of 0.5-3 cm. The guidance electrodes are energised by the voltage of some 30-1000 V although the voltage outside this range is also possible.

Figs. 11, 12a and 12b show photographs of the separation of cells on the fly ex-situ of the channel using embodiment of the device outlined in Fig. 1 and in Fig. 10. The cells are packed into droplets formed from the jet emitted from the microfluidic channel. Fig. 12a and 12b show photographs of the location along the jet where the jet splits into droplets, the point labelled by letter A in Fig. 10. This location is typically at the distance of some 50-5000 microns away from end point of the microfluidic channel. This distance can be easily controlled and adjusted in a very reproducible manner by changing the velocity of the jet, e.g. by changing the flow rate of the fluid in the channel. The greater the velocity and the greater the cross-sectional dimension of the microfluidic channel (width and height), the greater is that distance. Fig. 12a is a photograph of the device performing generation of smaller droplets at the rate of 33000 droplets per second. Fig. 12b is a photograph of the device performing generation of larger droplets at the rate of 27000 droplets per second.

The rate of the droplet generation is adjusted by the frequency of the actuation of the piezo element described in this document earlier. The frequency cannot be changed at will as there are favoured ranges of frequencies determined by the hydrodynamics of formation of microdrops from the continuous jet. These favoured ranges can be easily determined experimentally from the amplitude of the voltage applied to the piezo actuator necessary to generate a train of droplets: this voltage is lower in the favoured frequency regions. Fig. 11 shows photograph from a location position downstream with respect to the locations presented in Figs. 12a and 12b. In this experiment we separate three drops in a row from a continuous train of drops. The separation is done by the electric filed positioned substantially orthogonal to the trajectory of the droplets. The field is generated by the guidance electrodes. The guidance electrodes are marked by the numeral 26, 27 in Fig. 10 and the field is marked schematically by a tow-sided arrow. The reason why three droplets are taken in a row instead of one is to increase reliability and decrease the statistical error in the cell separation process: sometimes it is difficult to be certain as to the exact location of the cell detected in the detection zone with the precision of one droplet in the train of droplets. With separation of tree droplets instead of one, the statistical error is much reduced. One can see the droplets in the rest of the train are also affected by the charging electrodes 26, 27 as is evident from the fact that they do not follow the straight line but rather some of them are deviated leftward forming hook-like shapes on the photograph Fig. 11. These details are not important for the separation of cells, the important point is that the drops with the desired cells can be directed to a different destination point, which is to the right of the rest of the droplets with reference to Fig. 11.

The molecules/entities to be introduced into the cells can be introduced into the flow of cell-containing liquid or sheath fluid. Alternatively, these molecules/entities could be introduced via separate channel merging e.g. downstream from the electroporation zone or even downstream from the separation zone. Alternatively, these molecules/entities can be introduced into a destination container collecting the cells after the electroporation. In essence, both options can be valid: introduction of these into the flow alongside with the cells at some point of their movement along the microfluidic channel or introduction into a container collecting cell on the exit from the channel either directly on the microfluidic chip or outside of the microfluidic chip.

The cross section of the channel may change between any of the zones mentioned earlier. For example, the width of the microfluidic channel in may change between the detection zone and the electroporation zone or between the electroporation zone and the secondary detection zone or between the secondary detection zone and the separation zone. The change in the dimensions of the channels may or may not result in the change of the cross-section of the channel. For example, one may have a situation where the width of the channel increases by a factor of 2 and the height does not change thus resulting in the increase of the cross-sectional area of the channel by a factor of 2. In another embodiment the width could increase by a factor of 2 and the height could reduce by a factor or 2 thus resulting in no change of the cross-sectional area of channel. If the cross-sectional area changes by a factor of k, the linear flow velocity will change by a factor of k-1. Therefore, if the cross-sectional area of the channel changes, one needs to re-scale the linear velocity of the cells when as described above in calculation of the time delay it takes for the cells to travel from one zone to another one.

The microfluidic channel does not need to be straight. The channel can bend along the length once or several times and it can also have one or more abrupt turns along the channel.

The microchannel structure does not need to be planar. Different sections of the channel do not have to be positioned in the same plane. Three-dimensional structures are also possible. These will not be shown for brevity.

In our embodiments the width of the channels is in the range of 1 to 2000 microns, the height of the channels is in the range of 1 to 2000 microns. These figures are given as indications of the cross-sectional dimensions of the channel. One should keep in mind that although the rectangular cross-section of the channels is common, they do not have to be of rectangular or circular cross-section, and instead could have the cross section of e.g. a polygon-like or an ellipse-like shape. The length of the channel is typically in the range of 0.1 mm-500 mm although the dimensions outside this range are also possible. The pressure applied to the channels is in the range of 2 Bar.

In our device we could use a detection zone positioned downstream from the flow obstacle. The detection zone could be equipped with sensors to establish the condition of each cell. The cells with intact membrane will have different electric characteristics from the ones where the membrane was altered by the flow obstacle. We describe in detail the methods and apparatus for measurement of the status of the cell and in particular measurements of status of the cell membrane in the patent applications WO2017/182599 and WO2017/202932. For example, one could readily determine the size of the cells and establish if they are dead or alive on the basis of their electrical characteristics by variable frequency AC measurements. The device could also be equipped with the means for separating the cells following the procedure for the alteration of the cell membrane or following the procedure of the transfer of the biological material across the altered membrane (transfection). For the separation, the apparatus could be equipped with a separation zone downstream of the detection zone comprising a force generator configured to displace single cells in response to a single cell-specific parameter detected by the sensor. The examples of the force generators, the detection zone and the separation zones are given in patent applications WO2017/182599 and WO2017/202932.

Some of these features are described in detail in patent applications WO2017/182599 and WO2017/202932.

## Claims

1. A system for continuous high-throughput treatment of a population of cells, comprising:
a fluidic device comprising a microfluidic channel having an upstream detection zone comprising a detection electrode module configured to detect a change in electrical impedance across the microfluidic channel at the upstream detection zone corresponding to a cell passing the upstream detection zone and a cell treatment zone located downstream of the upstream detection zone and comprising a cell treatment module configured to treat the cell passing the cell treatment zone;
a pump fluidically coupled to the fluidic device and configured to pump the population of cells in a carrier liquid unidirectionally along the microfluidic channel; and
a processor operatively coupled to the detection electrode module and configured to calculate the velocity V of the cell passing the upstream detection zone based on the change in electrical impedance, and *transiently actuate* the cell treatment module when the cell reaches the cell treatment module based on the calculated velocity V of the cell and a distance D1 between the detection electrode module and the cell treatment module.

2. A system according to Claim 1, in which the detection electrode module comprises a first detection electrode pair configured to detect a first change in electrical impedance across the microfluidic channel corresponding to the cell passing the first detection electrode pair and a second detection electrode pair configured to detect a second change in electrical impedance across the microfluidic channel corresponding to the cell passing the second detection electrode pair, wherein the processor is configured to analyse the detected first and second changes in electrical impedance and determine the time period T1 it takes for the cell to pass from between the first and second detection electrode pairs, and calculate the velocity V of the cell based on the time period T1 and the distance D1 between the first and second detection electrode pairs.

3. A system according to Claim 1 or 2, in which the processor is configured to transiently actuate the cell treatment module for a treatment period T2 corresponding to the cell passing all or part of the cell treatment module.

4. A system according to any preceding Claim for transfection of cells, in which the cell treatment module comprises a cell electroporation module configured to electroporate cells passing the cell electroporation module by passing a cell electroporation voltage across the microfluidic channel.

5. A system according to Claim 4, in which the processor is configured to compare the change in electrical impedance detected by the detection electrode module corresponding to a cell passing the upstream detection zone module with a reference change in electrical impedance, calculate a cell electroporation parameter selected from amplitude of voltage or number or duration of the electroporation pulse based on the comparison, and actuate the cell electroporation module to apply the electroporation parameter to the cell.

6. A system according to any preceding Claim comprising a hydrodynamic cell focussing apparatus fluidically coupled to the microfluidic channel and configured to focus the population of cells into a single train of cells in the carrier liquid upstream of the upstream detection zone.

7. A system according to Claim 6, in which the cell hydrodynamic focussing apparatus is configured to focus the single train of cells asymmetrically in the microfluidic channel so that the cells are disposed closer to one electrode of a detection electrode module than a second electrode.

8. A system according to any preceding Claim, including a downstream detection zone disposed downstream of the cell treatment zone and comprising a detection electrode module configured to detect a downstream change in electrical impedance across the microfluidic channel at the downstream detection zone corresponding to a cell passing the downstream detection zone, wherein the processor is operatively coupled to the second detection electrode module.

9. A system according to Claim 8, in which the processor is configured to compare the downstream change in electrical impedance corresponding to a cell passing the downstream detection zone with a reference change in electrical impedance corresponding to a known cell electroporation status, and determine electroporation status of the cell based on the comparison.

10. A system according to Claim 8, in which the processor is configured to compare the downstream change in electrical impedance corresponding to a cell passing the downstream detection zone with an upstream change in electrical impedance detected by the upstream detection zone corresponding to the same cell passing the upstream detection zone, and determine electroporation status of the cell based on the comparison.

11. A system according to Claim 9 or 10, in which the electroporation status is selected from cell viability, successful cell electroporation, and unsuccessful cell electroporation.

12. A system according to Claim 8, in which the processor is configured to compare the downstream change in electrical impedance corresponding to a cell passing the downstream detection zone with a reference change in electrical impedance corresponding to a known cell electroporation status, and then actuate the cell electroporation module to modify the cell electroporation voltage or the duration of the cell electroporation voltage pulse applied across the microfluidic channel based on the comparison.

13. A system according to Claim 8, in which the processor is configured to compare the downstream change in electrical impedance corresponding to a cell passing the downstream detection zone with an upstream change in electrical impedance detected by the upstream detection zone corresponding to the same cell passing the upstream detection zone, and actuate the cell electroporation module to modify the cell electroporation voltage or the cell electroporation voltage pulse applied across the microfluidic channel based on the comparison.

14. A system according to any preceding Claim, in which the pump is configured to pump the population of cells and carrier fluid along the microfluidic channel with a linear flow velocity in the range of 0.05-2 m/s.

15. A continuous high-throughput method of treating a population of cells comprising the steps of
pumping the population of cells in a carrier liquid unidirectionally along a microfluidic channel having an upstream detection zone comprising a detection electrode module and a cell treatment zone located downstream of the detection zone comprising the cell treatment module;
actuating the detection electrode module to detect a change in electrical impedance across the microfluidic channel corresponding to a cell passing the upstream detection zone; calculating the velocity V of the cell passing the upstream detection zone based on the change in electrical impedance; and
*transiently actuating* the cell treatment module when the cell reaches the cell treatment module based on the calculated velocity V of the cell and a distance D1 between the detection electrode module and the cell treatment module.

## Patentansprüche

1. System zur kontinuierlichen Hochdurchsatz-Behandlung einer Population von Zellen, das Folgendes umfasst:
eine fluidische Vorrichtung, die einen mikrofluidischen Kanal umfasst, der Folgendes aufweist: eine vorgelagerte Detektionszone, die ein Detektionselektrodenmodul umfasst, das zur Detektion einer Änderung der elektrischen Impedanz über den mikrofluidischen Kanal an der vorgelagerten Detektionszone konfiguriert ist, die dem Passieren einer Zelle durch die vorgelagerte Detektionszone entspricht, und eine Zellbehandlungszone, die sich nachgelagert von der vorgelagerten Detektionszone befindet und ein Zellbehandlungsmodul umfasst, das zur Behandlung der Zelle, die die Zellbehandlungszone passiert, konfiguriert ist;
eine Pumpe, die fluidisch mit der fluidischen Vorrichtung verbunden ist und zum Pumpen der Population von Zellen in einer Trägerflüssigkeit in einer Richtung entlang des mikrofluidischen Kanals konfiguriert ist; und
einen Prozessor, der funktionsfähig mit dem Detektionselektrodenmodul verbunden ist und für Folgendes konfiguriert ist: Berechnen der Geschwindigkeit V der Zelle, die die vorgelagerte Detektionszone passiert, basierend auf der Änderung der elektrischen Impedanz, und *transientes Aktivieren* des Zellbehandlungsmoduls, wenn die Zelle das Zellbehandlungsmodul erreicht, basierend auf der berechneten Geschwindigkeit V der Zelle und einem Abstand D1 zwischen dem Detektionselektrodenmodul und dem Zellbehandlungsmodul.

2. System nach Anspruch 1, in dem das Detektionselektrodenmodul Folgendes umfasst: ein erstes Detektionselektrodenpaar, das zur Detektion einer ersten Änderung der elektrischen Impedanz über den mikrofluidischen Kanal konfiguriert ist, die dem Passieren der Zelle durch das erste Detektionselektrodenpaar entspricht, und ein zweites Detektionselektrodenpaar, das zur Detektion einer zweiten Änderung der elektrischen Impedanz über den mikrofluidischen Kanal konfiguriert ist, die dem Passieren der Zelle durch das zweite Detektionselektrodenpaar entspricht, wobei der Prozessor für Folgendes konfiguriert ist: Analyse der detektierten ersten und zweiten Änderung der elektrischen Impedanz und Bestimmung der Zeitdauer T1, die die Zelle benötigt, um zwischen dem ersten und zweiten Detektionselektrodenpaar zu passieren, und Berechnung der Geschwindigkeit V der Zelle basierend auf der Zeitdauer T1 und dem Abstand D1 zwischen dem ersten und zweiten Detektionselektrodenpaar.

3. System nach Anspruch 1 oder 2, in dem der Prozessor zur transienten Aktivierung des Zellbehandlungsmoduls für eine Behandlungsdauer T2 konfiguriert ist, die dem Passieren der Zelle durch das gesamte oder einen Teil des Zellbehandlungsmoduls entspricht.

4. System nach einem vorstehenden Anspruch zur Transfektion von Zellen, in dem das Zellbehandlungsmodul ein Zellelektroporationsmodul umfasst, das zur Elektroporation von Zellen, die das Zellelektroporationsmodul passieren, durch Anlegen einer Zellelektroporationsspannung über den mikrofluidischen Kanal konfiguriert ist.

5. System nach Anspruch 4, in dem der Prozessor für Folgendes konfiguriert ist:
Vergleich der Änderung der elektrischen Impedanz, die durch das Detektionselektrodenmodul detektiert wird, die dem Passieren einer Zelle durch das vorgelagerte Detektionszonenmodul entspricht, mit einer Referenzänderung der elektrischen Impedanz, Berechnung eines Zellelektroporationsparameters, der aus der Amplitude der Spannung oder Anzahl oder Dauer der Elektroporationspulse ausgewählt ist, basierend auf dem Vergleich, und Aktivierung des Zellelektroporationsmoduls, um den Elektroporationsparameter auf die Zelle anzuwenden.

6. System nach einem vorstehenden Anspruch, das einen hydrodynamischen Zellfokussierungsapparat umfasst, der fluidisch mit dem mikrofluidischen Kanal verbunden ist und zur Fokussierung der Population von Zellen zu einer einzigen Folge von Zellen in der Trägerflüssigkeit vorgelagert zu der vorgelagerten Detektionszone konfiguriert ist.

7. System nach Anspruch 6, in dem der hydrodynamische Zellfokussierungsapparat zur Fokussierung der einzigen Folge von Zellen asymmetrisch in dem mikrofluidischen Kanal konfiguriert ist, sodass die Zellen näher zu einer Elektrode eines Detektionselektrodenmoduls angeordnet sind als einer zweiten Elektrode.

8. System nach einem vorstehenden Anspruch, das eine nachgelagerte Detektionszone einschließt, die nachgelagert von der Zellbehandlungszone angeordnet ist und ein Detektionselektrodenmodul umfasst, das zur Detektion einer nachgelagerten Änderung der elektrischen Impedanz über den mikrofluidischen Kanal an der nachgelagerten Detektionszone konfiguriert ist, die dem Passieren einer Zelle durch die nachgelagerte Detektionszone entspricht, wobei der Prozessor funktionsfähig mit dem zweiten Detektionselektrodenmodul verbunden ist.

9. System nach Anspruch 8, in dem der Prozessor für Folgendes konfiguriert ist:
Vergleich der nachgelagerten Änderung der elektrischen Impedanz, die dem Passieren einer Zelle durch die nachgelagerte Detektionszone entspricht, mit einer Referenzänderung der elektrischen Impedanz, die einem bekannten Zellelektroporationszustand entspricht, und Bestimmung des Elektroporationszustands der Zelle basierend auf dem Vergleich.

10. System nach Anspruch 8, in dem der Prozessor für Folgendes konfiguriert ist:
Vergleich der nachgelagerten Änderung der elektrischen Impedanz, die dem Passieren einer Zelle durch die nachgelagerte Detektionszone entspricht, mit einer vorgelagerten Änderung der elektrischen Impedanz, die durch die vorgelagerte Detektionszone detektiert wird, die dem Passieren der gleichen Zelle durch die vorgelagerte Detektionszone entspricht, und Bestimmung des Elektroporationszustands der Zelle basierend auf dem Vergleich.

11. System nach Anspruch 9 oder 10, in dem der Elektroporationszustand aus Zellviabilität, erfolgreicher Zellelektroporation und erfolgloser Zellelektroporation ausgewählt ist.

12. System nach Anspruch 8, in dem der Prozessor für Folgendes konfiguriert ist:
Vergleich der nachgelagerten Änderung der elektrischen Impedanz, die dem Passieren einer Zelle durch die nachgelagerte Detektionszone entspricht, mit einer Referenzänderung der elektrischen Impedanz, die einem bekannten Zellelektroporationszustand entspricht, und dann Aktivierung des Zellelektroporationsmoduls zur Modifizierung der Zellelektroporationsspannung oder der Dauer des Zellelektroporationsspannungspulses, die/der über den mikrofluidischen Kanal angewendet wird, basierend auf dem Vergleich.

13. System nach Anspruch 8, in dem der Prozessor für Folgendes konfiguriert ist:
Vergleich der nachgelagerten Änderung der elektrischen Impedanz, die dem Passieren einer Zelle durch die nachgelagerte Detektionszone entspricht, mit einer vorgelagerten Änderung der elektrischen Impedanz, die durch die vorgelagerte Detektionszone detektiert wird, die dem Passieren der gleichen Zelle durch die vorgelagerte Detektionszone entspricht, und Aktivierung des Zellelektroporationsmoduls zur Modifizierung der Zellelektroporationsspannung oder des Zellelektroporationsspannungspulses, die/der über den mikrofluidischen Kanal angewendet wird, basierend auf dem Vergleich.

14. System nach einem vorstehenden Anspruch, in dem die Pumpe zum Pumpen der Population von Zellen und Trägerflüssigkeit entlang dem mikrofluidischen Kanal mit einer linearen Fließgeschwindigkeit in dem Bereich von 0,05-2 m/s konfiguriert ist.

15. Kontinuierliches Hochdurchsatz-Verfahren zur Behandlung einer Population von Zellen, das die folgenden Schritte umfasst:
Pumpen der Population von Zellen in einer Trägerflüssigkeit in einer Richtung entlang eines mikrofluidischen Kanals, der Folgendes aufweist: eine vorgelagerte Detektionszone, die ein Detektionselektrodenmodul umfasst, und eine Zellbehandlungszone, die sich nachgelagert von der Detektionszone befindet, die das Zellbehandlungsmodul umfasst,
Aktivieren des Detektionselektrodenmoduls zur Detektion einer Änderung der elektrischen Impedanz über den mikrofluidischen Kanal, die dem Passieren einer Zelle durch die vorgelagerte Detektionszone entspricht,
Berechnen der Geschwindigkeit V der Zelle, die die vorgelagerte Detektionszone passiert, basierend auf der Änderung der elektrischen Impedanz, und
*transientes Aktivieren* des Zellbehandlungsmoduls, wenn die Zelle das Zellbehandlungsmodul erreicht, basierend auf der berechneten Geschwindigkeit V der Zelle und einem Abstand D1 zwischen dem Detektionselektrodenmodul und dem Zellbehandlungsmodul.

## Revendications

1. Système destiné au traitement continu à haut débit d'une population de cellules, comprenant :
un dispositif fluidique comprenant un canal microfluidique ayant une zone de détection en amont comprenant un module d'électrodes de détection configuré pour détecter un changement d'impédance électrique à travers le canal microfluidique au niveau de la zone de détection en amont correspondant à une cellule passant la zone de détection en amont et une zone de traitement cellulaire située en aval de la zone de détection en amont et comprenant un module de traitement cellulaire configuré pour traiter la cellule passant la zone de traitement cellulaire ;
une pompe couplée de manière fluidique au dispositif fluidique et configurée pour pomper la population de cellules dans un liquide porteur de manière unidirectionnelle le long du canal microfluidique ; et
un processeur couplé de manière opérationnelle au module d'électrodes de détection et configuré pour calculer la vitesse V de la cellule passant la zone de détection en amont sur la base du changement d'impédance électrique, et pour *actionner de manière transitoire* le module de traitement cellulaire lorsque la cellule atteint le module de traitement cellulaire sur la base de la vitesse V calculée de la cellule et d'une distance D1 entre le module d'électrodes de détection et le module de traitement cellulaire.

2. Système selon la revendication 1, dans lequel le module d'électrodes de détection comprend une première paire d'électrodes de détection configurée pour détecter un premier changement d'impédance électrique à travers le canal microfluidique correspondant à la cellule passant la première paire d'électrodes de détection et une deuxième paire d'électrodes de détection configurée pour détecter un deuxième changement d'impédance électrique à travers le canal microfluidique correspondant à la cellule passant la deuxième paire d'électrodes de détection, où le processeur est configuré pour analyser le premier et le deuxième changements détectés d'impédance électrique et pour déterminer la période de temps T1 nécessaire pour que la cellule passe entre la première puis la deuxième paire d'électrodes de détection, et calculer la vitesse V de la cellule sur la base de la période de temps T1 et de la distance D1 entre la première et la deuxième paire d'électrodes de détection.

3. Système selon la revendication 1 ou 2, dans lequel le processeur est configuré pour actionner de manière transitoire le module de traitement cellulaire pendant une période de traitement T2 correspondant à la cellule passant la totalité ou une partie du module de traitement cellulaire.

4. Système selon l'une quelconque des revendications précédentes destiné à la transfection de cellules, dans lequel le module de traitement cellulaire comprend un module d'électroporation cellulaire configuré pour l'électroporation des cellules passant le module d'électroporation cellulaire, en faisant passer une tension d'électroporation cellulaire à travers le canal microfluidique.

5. Système selon la revendication 4, dans lequel le processeur est configuré pour comparer le changement d'impédance électrique détecté par le module d'électrodes de détection correspondant à une cellule passant le module de zone de détection en amont avec un changement de référence d'impédance électrique, calculer un paramètre d'électroporation cellulaire choisi parmi l'amplitude de la tension ou le nombre ou la durée de l'impulsion d'électroporation sur la base de la comparaison, et actionner le module d'électroporation cellulaire pour appliquer le paramètre d'électroporation à la cellule.

6. Système selon l'une quelconque des revendications précédentes, comprenant un appareil de focalisation hydrodynamique des cellules couplé de manière fluidique au canal microfluidique et configuré pour focaliser la population de cellules en un train unique de cellules dans le liquide porteur en amont de la zone de détection en amont.

7. Système selon la revendication 6, dans lequel l'appareil de focalisation hydrodynamique des cellules est configuré pour focaliser le train unique de cellules de manière asymétrique dans le canal microfluidique de façon à ce que les cellules soient disposées plus près d'une électrode d'un module d'électrodes de détection que d'une deuxième électrode.

8. Système selon l'une quelconque des revendications précédentes, comprenant une zone de détection en aval disposée en aval de la zone de traitement cellulaire et comprenant un module d'électrodes de détection configuré pour détecter un changement en aval d'impédance électrique à travers le canal microfluidique au niveau de la zone de détection en aval correspondant à une cellule passant la zone de détection en aval, où le processeur est couplé de manière opérationnelle au deuxième module d'électrodes de détection.

9. Système selon la revendication 8, dans lequel le processeur est configuré pour comparer le changement en aval d'impédance électrique correspondant à une cellule passant la zone de détection en aval avec un changement de référence d'impédance électrique correspondant à un état d'électroporation cellulaire connu, et déterminer l'état d'électroporation de la cellule sur la base de la comparaison.

10. Système selon la revendication 8, dans lequel le processeur est configuré pour comparer le changement en aval d'impédance électrique correspondant à une cellule passant la zone de détection en aval avec un changement en amont d'impédance électrique détecté par la zone de détection en amont correspondant à la même cellule passant la zone de détection en amont, et déterminer l'état d'électroporation de la cellule sur la base de la comparaison.

11. Système selon la revendication 9 ou 10, dans lequel l'état d'électroporation est choisi parmi la viabilité cellulaire, l'électroporation cellulaire réussie et l'électroporation cellulaire non réussie.

12. Système selon la revendication 8, dans lequel le processeur est configuré pour comparer le changement en aval d'impédance électrique correspondant à une cellule passant la zone de détection en aval avec un changement de référence d'impédance électrique correspondant à un état d'électroporation cellulaire connu, puis actionner le module d'électroporation cellulaire afin de modifier la tension d'électroporation cellulaire ou la durée de l'impulsion de tension d'électroporation cellulaire appliquée à travers le canal microfluidique, sur la base de la comparaison.

13. Système selon la revendication 8, dans lequel le processeur est configuré pour comparer le changement en aval d'impédance électrique correspondant à une cellule passant la zone de détection en aval avec un changement en amont d'impédance électrique détecté par la zone de détection en amont correspondant à la même cellule passant la zone de détection en amont, et actionner le module d'électroporation cellulaire afin de modifier la tension d'électroporation cellulaire ou l'impulsion de tension d'électroporation cellulaire appliquée à travers le canal microfluidique, sur la base de la comparaison.

14. Système selon l'une quelconque des revendications précédentes, dans lequel la pompe est configurée pour pomper la population de cellules et le liquide porteur le long du canal microfluidique selon une vitesse d'écoulement linéaire dans la plage de 0,05-2 m/s.

15. Méthode continue à haut débit de traitement d'une population de cellules, comprenant les étapes
de pompage de la population de cellules dans un liquide porteur de manière unidirectionnelle le long d'un canal microfluidique ayant une zone de détection en amont comprenant un module d'électrodes de détection et une zone de traitement cellulaire située en aval de la zone de détection comprenant le module de traitement cellulaire ;
d'actionnement du module d'électrodes de détection pour détecter un changement d'impédance électrique à travers le canal microfluidique correspondant à une cellule passant la zone de détection en amont ;
de calcul de la vitesse V de la cellule passant la zone de détection en amont, sur la base du changement d'impédance électrique ; et
*d'actionnement, de manière transitoire,* du module de traitement cellulaire lorsque la cellule atteint le module de traitement cellulaire sur la base de la vitesse V calculée de la cellule et d'une distance D1 entre le module d'électrodes de détection et le module de traitement cellulaire.
